# EUROPÄISCHE PATENTSCHRIFT

(11) **EP 1 667 743 B1**
(45) Veröffentlichungstag und Bekanntmachung des Hinweises auf die Patenterteilung: **02.01.2008**
(21) Anmeldenummer: 04786896.3
(22) Anmeldetag: 29.09.2004
(51) Int. Cl.: A61L 27/00

(54) **BIOKOMPATIBLE, BIOSTABILE BESCHICHTUNG VON MEDIZINISCHEN OBERFLÄCHEN**
BIOCOMPATIBLE, BIOSTABLE COATING OF MEDICAL SURFACES
REVETEMENT BIOCOMPATIBLE, BIOSTABLE DE SURFACES MEDICALES

(30) Priorität: 29.09.2003 DE 10345132; 03.11.2003 US 516295 P; 28.04.2004 DE 102004020856; 17.05.2004 US 571582 P
(43) Veröffentlichungstag der Anmeldung: 14.06.2006
(73) Patentinhaber: Hemoteq AG, 52146 Würselen (DE)
(72) Erfinder: HORRES, Roland, 52223 Stolberg (DE); HOFFMANN, Michael, 52249 Eschweiler (DE); FAUST, Volker, 52080 Aachen (DE); HOFFMANN, Erika, 52249 Eschweiler (DE); DI BIASE, Donato, 52080 Aachen (DE)
(74) Vertreter: Arth, Hans-Lothar
(86) Internationale Anmeldenummer: PCT/DE2004/002184
(87) Internationale Veröffentlichungsnummer: WO 2005/032611

(56) Entgegenhaltungen:
- EP-A- 1 026 190
- US-A- 4 179 757
- US-A1- 2003 171 804
- US-B1- 6 500 549

## Beschreibung

Die Erfindung betrifft hemokompatible medizinische Oberflächen mit einer biokompatiblen, biostabilen Beschichtung aus Polysulfonen oder/und Polysulfonderivaten bzw. Copolymeren mit Polysulfon, welche mindestens einen antiproliferativen, antiinflammatorischen, antiphlogistischen und/oder antithrombotischen Wirkstoff enthält und/oder von Wirkstoff bedeckt wird, Verfahren zur Herstellung dieser Oberflächen sowie ihre Verwendung in Form von Langzeit-Implantaten, insbesondere Stents zur Verhinderung von Restenose.

In den letzten Jahren hat das Einsetzen von Stents bei der Ballondilatation von verschlossenen Blutgefäßen immer weitere Verbreitung gefunden. Obwohl Stents das Risiko eines erneuten Gefäßverschlusses verkleinern, sind sie bisher nicht in der Lage, solche Restenosen vollständig zu verhindern.

Eine genaue begriffliche Beschreibung der Restenose ist in der Fachliteratur nicht aufzufinden. Die am häufigsten verwendete morphologische Definition der Restenose ist diejenige, die nach erfolgreicher PTCA (perkutane transluminale Koronarangioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.

Für die durch den Stent verursachte Restenose gibt es zwei verschiedene Ursachen:
a.) In der ersten Zeit nach der Implantation ist die Oberfläche des Stents dem Blut direkt ausgesetzt und es kann aufgrund der nun vorhandenen Fremdoberfläche zu einer akuten Thrombose kommen, die das Blutgefäß wieder verschließt.
b.) Mit Implantation des Stent werden Gefäßverletzungen verursacht, die neben der in oben genannten Thrombose, ebenfalls Entzündungsreaktionen hervorrufen, die für den Heilungsprozeß in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einem erneuten Verschluß des Gefäßes aufgrund unkontrollierten Wachstums führen.
c.) Nach einigen Wochen beginnt der Stent in das Gewebe des Blutgefäßes einzuwachsen. Das heißt, daß der Stent vollständig von glatten Muskelzellen umhüllt wird und keinen Kontakt mehr zum Blut hat. Diese Vernarbung kann zu stark ausgeprägt sein (Neointimahyperplasie) und dazu führen, daß nicht nur die Stentoberfläche bedeckt, sondern der ganze Innenraum des Stents verschlossen wird.

Man hat vergeblich versucht, das Problem der Restenose durch die Beschichtung der Stents mit Heparin zu lösen (J. Whörle et al, European Heart Journal (2001) 22 1808-1816). Heparin adressiert als Antikoagulanz jedoch nur die erstgenannte Ursache und kann darüber hinaus nur in Lösung seine volle Wirkung entfalten. Dieses erste Problem läßt sich mittlerweile medikamentös durch die Gabe von Antikoagulantien fast vollständig vermeiden. Das zweite und dritte Problem versucht man zur Zeit zu lösen, indem man das Wachstum der glatten Muskelzellen lokal am Stent hemmt. Das wird z.B. mit radioaktiven Stents oder mit Stents versucht, welche pharmazeutische Wirkstoffe enthalten.

So offenbart US-A-5 891 108 beispielsweise einen hohl ausgeformten Stent, welcher in seinem Inneren pharmazeutische Wirkstoffe enthalten kann, welche durch eine Vielzahl von Öffnungen im Stent freigesetzt werden. EP-A-1 127 582 beschreibt hingegen einen Stent, der auf seiner Oberfläche Einbuchtungen von 0,1 - 1 mm Tiefe und 7-15 mm Länge aufweist, welche zur Aufnahme eines Wirkstoffes geeignet sind. Diese Wirkstoffreservoire setzen, vergleichbar der Öffnungen in dem hohlen Stent, den enthaltenen pharmazeutischen Wirkstoff punktuell in hoher Konzentration und über einen relativ langen Zeitraum frei, was aber dazu führt, daß die glatten Muskelzellen nicht mehr oder nur sehr verzögert in der Lage sind, den Stent zu umhüllen. Daher ist der Stent viel länger dem Blut ausgesetzt, was wieder vermehrt zu Gefäßverschlüssen durch Thrombosen führt (Liistro F., Colombo A., Late acute thrombosis after Paclitaxel eluting stent implantation. Heart (2001) 86 262-4).
Ein Lösungsversuch für dieses Problem stellt die Phosphorylcholinbeschichtung von Biocompatibles (WO 0101957) dar, indem hier Phosphorylcholin, eine Zellmembrankomponente der Erythrocyten, als Bestandteil der aufgebrachten nicht bioabbaubaren Polymerschicht auf dem Stent eine nichtthrombogene Oberfläche erzeugen soll. Dabei wird der Wirkstoff abhängig vom Molekulargewicht von dieser polymerhaltigen Phosphorylcholinschicht absorbiert oder auf der Oberfläche adsorbiert.

Des weiteren offenbart EP 1 026 190 A ein Poly(alkylarylether)sulfon-Copolymer mit guten antithrombogenen Eigenschaften zur Herstellung oder Beschichtung von Medizinprodukten.

US 2003/171804 A beschreibt einen Stent mit einer Beschichtung aus einem Trägerpolymer, an welches Perfluoralkylketten gebunden sind. Unter anderem können auch Polysulfone als Trägerpolymer eingesetzt werden.

US 6,500,549 B ist auf medizinische Artikel gerichtet, welche eine Beschichtung eines Copolymers aus einem filmbildenden und einem hydrophoben Polymer aufweisen. Als medizinische Artikel werden auch Stents erwähnt und als filmbildendes Polymer können unter anderem auch Polysulfone wie beispielsweise Polyethersulfone eingesetzt werden.

Keines der vorgenannten Dokumente erwähnt jedoch die Anwesenheit eines antiproliferativen, antiinflammatorischen, antiphlogistischen und/oder antithrombotischen Wirkstoffs sowie die Funktion der Polysulfonbeschichtung als Wirkstoffträger.

Aufgabe der vorliegenden Erfindung ist es, ein Medizinprodukt mit einer hämokompatiblen Oberfläche sowie ein Herstellungsverfahren für dieses Medizinprodukt mit der hämokompatiblen Oberfläche bereitzustellen, welches zur Freisetzung eines antiproliferativen, antiinflammatorischen, antiphlogistischen und/oder antithrombotischen Wirkstoffs geeignet ist.
Insbesondere soll die hämokompatible Oberfläche des Medizinprodukts ein kontinuierliches und kontrolliertes Einwachsen des Medizinprodukts in die Gefäßwand erlauben.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung, den Figuren sowie den Beispielen.

Die vorliegende Erfindung betrifft hemokompatible Medizinprodukte, deren Oberfläche(n) zumindest teilweise mit mindestens einer biostabilen Schicht aus Polysulfon beschichtet ist(sind), worauf, worunter und/oder worin sich mindestens ein antiproliferativer, antiinflammatorischer, antiphlogistischer und/oder antithrombotischer Wirkstoff befindet.
Es hat sich überraschend herausgestellt, dass die Beschichtung von medizinischen Oberflächen, die in permanentem Blutkontakt stehen, mit Polysulfon, Polyethersulfon und/oder Polyphenylsulfon und deren Derivate ein äußerst gut geeigneter biokompatibler Träger für Wirkstoffe darstellt. Durch Zumischung von hydrophilen biokompatiblen Polymeren oder durch die Verwendung von Polysulfonen mit ambivalenten Eigenschaften, d.h. mit lipophilen und hydrophilen Resten läßt sich die Porengröße der Polysulfonmatrix so variieren, dass hierüber eine Vielzahl von Möglichkeiten hinsichtlich der verwendeten Wirkstoffe, ihrer einsetzbaren Menge als auch der gewünschten Freisetzungsrate ermöglicht werden. Insbesondere kann die Elutionskinetik des mindestens einen Wirkstoffes über die Porengröße in der biostabilen Schicht reguliert werden. Die Porengröße wird wiederum durch Art und Menge des verwendeten hydrophilen Polymeren bzw. der Menge an Iiopophilen und lipophoben Gruppen im Polysulfon oder Polysulfongemisch bestimmt. Neben dem Einfluß des zugefügten hydrophilen Polymeren wirkt sich die Addition von geringen Mengen Wasser (oder auch eben Essigsäureethylester) in der Beschichtungslösung auf die späteren Eigenschaften des wirkstoffbeladenen beschichteten Implantats aus. Die Einstellung der Beladungsverteilung, der Release-Eigenschaften (als Funktion der Zeit und der eluierten Wirkstoffmenge) und die Sprüheigenschaften der Beschichtungslösung wird entscheidend durch die definierte Zugabe von Wasser (oder auch eben Essigsäureethylester oder anderen weiter unten beschriebenen Additiven) in die Sprühlösung geprägt.

Es hat sich ebenso als vorteilhaft erwiesen, dass die Verwendung von Stickstoff als Trägergas zur Sprühbeschichtung zu einer Beladung der wirkstoffenthaltenden Polymerschicht mit Stickstoff führt, dass in der Schicht verbleibt und hier in seiner Eigenschaft als Schutzgas für die Unversehrtheit des Wirkstoffs sorgt. Damit wird die Haltbarkeit des Wirkstoffes in unverändert wirksamer Form dauerhaft gewährleistet.

Die Modifizierung des Polysulfongrundgerüsts durch polymeranaloge Reaktionen wie die Darstellung neuer Polysulfoncopolymere (z.B. als Polysulfonblockcopolymere, oder in statistischer Verteilung) hat Auswirkung auf das physikalische Verhalten der entstehenden Polymere, worüber sich Eigenschaften des Polymeren steuern lassen, und entweder in Kombination mit den unmodifizierten Polymeren oder allein als neues hämokompatibles Beschichtungsmaterial einsetzbar sind. So läßt sich über die Umsetzung von Polysulfoncopolymere mit 4,4'-Bis(hydroxylphenyl)pentansäure (BPA) ein carboxylgruppenhaltiges Polyethersulfon darstellen, was zu einer deutlichen Hydrophilie des Polymeren führt. Die Eigenschaften des hydrophilen Polysulfons lassen wie oben bereits erwähnt ebenfalls als hydrophiler Polymerzusatz zum unmodifizierten Polysulfon nutzen. Über die Einstellung des Modifizierungsgrades wird der Hydrophiliegrad beeinflusst, so dass ein Polymermolekül entsteht, in der jede Kette unmodifizierte und modifizierte Bereiche enthält und so in sich selbst hydrophobe und hydrophile Eigenschaften vereinigt, die dem Polymeren auch eine veränderte räumliche Anordnung der Kettensegmente, der sogenannten Sekundärstruktur, verleihen. Es ist daher bevorzugt, ein Polysulfon für die Beschichtung zu verwenden, welches hydrophile Bereiche und hydrophobe Bereiche aufweist. Derartige Polysulfone können hergestellt werden, indem ein Polysulfon nach der Polymerisation durch polymeranaloge Reaktionen mit hydrophilen Seitengruppen oder funktionellen Gruppen versehen wird, sofern das Polymere selbst hydrophob ist oder umgekehrt ein hydrophiles Polysulfon mit hydrophoben Seitengruppen oder funktionellen Gruppen versehen wird. Bei dieser bevorzugten Ausführungsform werden die hydrophilen und hydrophoben Eigenschaften in einem Polymermolekül vereinigt, in der Regel mit einer statistischen Verteilung, da die polymeranalogen Reaktionen mit einer statistischen Verteilung ablaufen. Ferner können solche Systeme aus hydrophilem Polysulfon mit hydrophobem Polysulfon durch statistische Polymerisation von mindestens einem hydrophilen Monomeren und mindestens einem hydrophoben Monomeren hergestellt werden. Hierdurch ergeben sich ähnliche Strukturen wie bei der vorgenannten Ausführungsform der nachträglichen Modifizierung durch polymeranaloge Reaktionen. Eine dritte Ausführungsform besteht in der Blockcopolymerisation von mindestens einem hydrophilen Sulfonblockpolymeren mit mindestens einem hydrophoben Sulfonblockpolymeren zu einem Polysulfon, welches die hydrophilen und hydrophoben Eigenschaften jeweils in den einzelnen Blöcken aufweist. Eine weitere Variante besteht darin, mindestens ein hydrophiles Monomer in einer alternierenden Copolymerisation mit mindestens einem hydrophoben Monomer umzusetzen. Hierbei sind die hydrophilen und hydrophoben Eigenschaften im erhaltenen Polysulfon alternierend in der Polymerkette verteilt. Ferner kann bei der erfindungsgemäßen Beschichtung eine Mischung aus mindestens einem hydrophilen Polysulfon mit mindestens einem hydrophoben Polysulfon eingesetzt werden. Hierbei sind die hydrophilen und hydrophoben Eigenschaften nicht in einem Polymermolekül vereinigt, finden sich aber in der Beschichtung wieder und ergeben die gleichen Effekte wie bei den vorgenannten Ausführungsformen.

Für die Herstellung der Polysulfone eignen sich sämtliche dem Fachmann bekannte Polymerisationsreaktionen, wie beispielsweise radikalische, anionische, kationische oder thermische Polymerisation. Beispiele für die vorgenannten Polysulfone sowie Möglichkeiten zu deren Herstellung werden weiter unter beschrieben.

Zudem besteht die Möglichkeit eingeführte funktionelle Gruppen, beispielsweise die Carboxylgruppe, zu derivatisieren (Macrom. Chem. Phys. 1994, 195, 1709). So läßt sich z.B. durch Einführung von fluorierten Verbindungen (ohne weiteres die Hydrophobie des Wirkstoffes über die hydrophoben Eigenschaften des eingesetzten Polymeren hinaus erhöhen (Coll. Polym. Sci. 2001, 279, 727). Es lassen sich über die Einführung von funktionellen Gruppen Propfcopolymere herstellen, wobei die Seitenketten nun aus anderen Struktureinheiten bestehen als die Hauptkette. Dazu lassen sich biokompatible biostabile und bioabbaubare Polymere einsetzen.

Die funktionellen Gruppen sind ebenfalls zu einer hydrolyselabilen Anbindung von Wirkstoffen zu verwenden. Der Wirkstoff wird in Folge dessen ebenfalls in einer über die Hydrolyse und in Abhängigkeit von der Bindungsart (Thioesterbindung, Esterbindung) kontrollierten Form abgegeben. Hier besteht der Vorteil in der Möglichkeit, die Elution des Wirkstoffes so zu kontrollieren, dass die Releasekurve einen anderen Verlauf nimmt und sich mit dem Implantat Adaptionen an viele verschiedene Krankheitsverläufe mit diversen Ansprüchen an die Wirkstoffkonzentration in Abhängigkeit von der Zeit erreichen lassen. Eine Variation besteht in der kovalenten Anbindung von desulfatiertem und N-reacetyliertem Heparin und/oder N-carboxymethyliertem und/oder partiell N-acetyliertem Chitosan an die Polymerkette, womit die Hämokompatibilität des Polymeren mit Hilfe der athrombogenen Verbindung verbessert wird.

Über die Möglichkeit des Aufbaus von mindestens zwei Schichten des in seiner Zusammensetzung variierbaren Polymers wie auch in der Variation der Additive läßt sich zudem eine schichtabhängige Differenzierung bezüglich der eingesetzten Wirkstoffe als auch bezüglich der Konzentration vornehmen. Diese Adaptionsfähigkeit zeichnet die Polysulfonmatrix als ein universell einsetzbares biostabiles Beschichtungsmaterial zur Verhinderung der Restenose aus.

Zur Einstellung der Porengröße und damit der Wirkstoffmenge in der Polysulfonmatrix können als Additive nicht nur hydrophile Polymere, sondern auch Mineralien und selbst Wasser verwendet werden. Die Porengröße steuert zum einen die Freisetzungskinetik des mindestens einen antiproliferativen, antiinflammatorischen, antiphlogistischen und/oder antithrombotischen Wirkstoffs als auch in bestimmten Ausführungsformen die Menge an Wirkstoff, welche in eine Polysulfonbeschichtung eingebracht bzw. aufgebracht werden kann, da die Poren im Polysulfon als Wirkstoffreservoir dienen können.

Zur Erzeugung von Poren in der Polysulfonmatrix beim Einsatz dieser Additive können bzw. müssen unterschiedliche Strategien verfolgt werden.

Prinzipiell verläuft die Erzeugung von Poren derart, dass auf das zu beschichtende Medizinprodukt die Additive mit dem matrixbildenden Polysulfon gemeinsam nach einem geeigneten Verfahren aufgebracht werden. Hierbei bilden sich in Abhängigkeit von den Unterschieden in der Hydrophilie der eingesetzten Additive als auch des matrixbildenden Polysulfons in ihrer Größe steuerbare homogene Kompartimente des Additivs. Die Anzahl dieser homogenen Kompartimente pro Volumeneinheit der Polysulfonmatrix ist über die prozentual zugesetzte Menge des Additivs steuerbar.

Als Additive können im einzelnen Aminosäuren, Polyaminosäuren, hydrophile Polymere, Saccharide, Oligosaccharide, Polysaccharide, Oligopeptide, Polyvinylpyrrolidon, Polyethylenimin, Glycerin, Polyether, Glykol, Mineralien und Wasser verwendet werden.

Im Fall der Aminosäuren werden die genetisch codierten sauren Aminosäuren Asparaginsäure, Glutaminsäure; die neutralen Aminosäuren Alanin, Asparagin, Cystein, Glutamin, Glycin, Isoleucin, Leucin, Methionin, Phenylalanin, Prolin, Serin, Threonin, Tryptophan, Tyrosin, Valin; und die basischen Aminosäuren Arginin, Histidin, Lysin; sowie die nicht genetisch codierten Aminosäuren Ornithin und Taurin bevorzugt. Insbesondere werden die Vertreter der L-Reihe dieser Aminosäuren bevorzugt. Ferner werden die Vertreter der D-Reihe dieser Aminosäuren sowie D,L-Mischungen einer Aminosäure als auch D,L-Mischungen mehrerer Aminosäuren bevorzugt.

Im Fall der Polyaminosäuren werden die Aminosäuren Poly-L-Asparaginsäure, Poly-L-Glutaminsäure, Poly-L-Alanin, Poly-L-Asparagin, Poly-L-Cystein, Poly-L-Glutamin, Poly-L-Glycin, Poly-L-Isoleucin, Poly-L-Leucin, Poly-L-Methionin, Poly-L-Phenylalanin, Poly-L-Prolin, Poly-L-Serin, Poly-L-Threonin, Poly-L-Tryptophan, Poly-L-Tyrosin, Poly-L-Valin, Poly-L-Arginin, Poly-L-Histidin, Poly-L-Lysin sowie Poly-Ornithin und Poly-Taurin bevorzugt. Ferner sind auch Vertreter der D-Reihe dieser Polyaminosäuren sowie D,L-Mischungen einer Polyaminosäure als auch D,L-Mischungen mehrerer Polyaminosäuren geeignet.

Im Fall der hydrophilen Polymere werden globulare Moleküle wie organische Nanopartikel, Sternpolymere, Dendrimere und/oder hochverzweigte Polymere bevorzugt.

Im Fall der Mineralien werden die Carbonate, Chlorate, Phosphate und Sulfate der Kationen Natrium, Calcium, Kalium und/oder Magnesium bevorzugt.

Zur Ausbildung der Porenstruktur werden diese Kompartimente anschließend aus der Polysulfonmatrix entfernt. Zurück bleibt die dreidimensionale Struktur mit dem vorbestimmten Grad der Porosität, die dann mit dem Wirkstoff "befüllt" werden kann.

Im folgenden werden drei bevorzugte Systeme zur Erzeugung der Porenstruktur kurz anhand der Additivklassen Polymer, Mineral und Wasser beschrieben.

### System 1: Polymer

Als polymere Additive werden z.B. spezielle hochverzweigte Polyester mit thermolabilen Triazengruppen in der Hauptkette eingesetzt. Das molekulardispergierte hochverzweigte Polymer wird in die Polysulfonmatrix eingebaut. Die anschließende thermische Behandlung des Systems zersetzt den hochverzweigten Porenbildner in flüchtige Zersetzungsprodukte unter Bildung einer entsprechenden nanoporösen Polymerschicht. Polysulfone zeichnen sich unter anderem durch ihre Temperaturbeständigkeit und hohe Dimensionsstabilität aus, wodurch diese Vorgehensweise durchaus anwendbar ist. Darüber hinaus kann diese thermische Behandlung mit dem Schritt der Sterilisation gekoppelt werden, was zu einem effizienten Verfahren führt.

### System 2: Mineral

Als mineralisches Additiv wird z.B. die physiologisch unbedenkliche Verbindung Calciumcarbonat eingesetzt. Die Polysulfonmatrix besteht aus doppelthydrophilen Blockcopolymeren. Diese doppelthydrophilen Polysulfonblockcopolymere umfassen einen hydrophilen Block, der mit dem mineralischen Additiv nicht wechselwirkt und einen zweiten Polyelektrolytblock, der mit den Oberflächen des mineralischen Additivs stark wechselwirkt. Diese Blockcopolymere wirken bei der Kristallisation von Calciumcarbonat wachstumsmodifizierend. Die resultierenden mineralischen Kompartimente besitzen eine annähernd ovale, hantel- oder kugelförmige Gestalt. Wegen der ausgezeichneten Resistenz der Polysulfone gegenüber aggressiven Chemikalien sowie der Hydrolysebeständigkeit können die mineralischen Additive im Säurebad vollständig entfernt werden. Zurück bleibt die gewünschte nanoporöse Struktur der Polysulfonmatrix.

### System 3: Wasser

Als fluides Additiv kommt im Falle der Beschichtung des Medizinprodukts mit polaren Wirkstoffen als einfachste Lösung Wasser in Betrachtung. Bei der Anwendung des Sprühverfahrens liegt das Polysulfon in einem organischen Lösungsmittel wie z.B. Chloroform vor. Die mit Polysulfon gesättigte Chloroformlösung ist für die weitere Aufnahme des Wirkstoffs nur bedingt in der Lage. Somit löst sich der Wirkstoff überwiegend in der wässrigen Phase, die nach der Auftragung auf die Oberfläche des Medizinprodukts bedingt durch die Phasenseparation Kompartimente bildet. Anschließend kann das Wasser aus diesen Kompartimenten beispielsweise mit Hilfe der Gefriertrocknung vollständig aus dem System entfernt werden. Zurück bleiben wirkstoffbeladene nanoporöse Strukturen. Die Wirkstoffkonzentration der Poren kann in nachfolgenden Schritten mit in Wasser gelöstem Wirkstoff und bevorzugt anschließender Gefriertrocknung erhöht werden. Bei den bisherigen Verfahren wurde zusammen mit dem Polysulfon auch der Wirkstoff in Chloroform gelöst. Die folgende Aufkonzentrierung des Wirkstoffs erfolgte ebenfalls aus einer Chloroformlösung. Weil das Chloroform aber nie zu 100 % aus der Schicht entfernt werden kann, konzentriert sich das Chloroform zunehmend in fertigen Endprodukt, was zu einer unnötigen Belastung des Patienten führt. Mit der Verwendung von Wasser als Wirkstoffträger wird nur einmal Chloroform für die Aufbringung der Polysulfonmatrix verwendet und die Belastung auf ein Minimum reduziert.

Zur Zubereitung einer Sprühlösung enthaltend mindestens ein Polysulfon und mindestens einen antiproliferativen, antiinflammatorischen, antiphlogistischen und/oder antithrombotischen Wirkstoff eignen sich zudem bevorzugt Lösungsmittel, welche leicht verdunsten, d.h. flüchtig sind wie beispielsweise Chloroform, Dichlormethan, Tetrahydrofuran, Aceton, Methanol, Ethanol, Isopropanol, Diethylether und Essigsäureethylester und welche zudem mit Wasser gesättigt oder mit einem bestimmten Wassergehalt hergestellt werden können. Dabei sind Wassergehalte von 1,6 - 15%, bevorzugt 2,1 - 10%, weiter bevorzugt 2,6 - 7,9% und insbesondere bevorzugt 3,3 - 6,8% geeignet. Ferner ist bevorzugt, wenn organisches Lösungsmittel, Wasser, Polysulfon und Wirkstoff eine homogene Lösung bilden.

Über die Bildung von Copolymeren läßt sich ebenfalls die Hydrophilie bzw. Hydrophobie des Polysulfons variieren. So kann man zum Beispiel Polysulfoncopolymere mit 4,4'-Bis(hydroxylphenyl)pentansäure (BPA) synthetisieren, so dass auf diese Weise Carboxylseitengruppen eingeführt werden, die die Hydrophobie des Polysulfon-Grundgerüstes senken. Zudem besteht nun die Möglichkeit eingeführte funktionelle Gruppen beispielsweise die Carboxylgruppe zu derivatisieren (Macrom. Chem. Phys. 195 (1994), 1709; Coll. Polym. Sci. 279 (2001), 727).

Über die Möglichkeit des Aufbaus von mindestens zwei Schichten des in seiner Zusammensetzung variierbaren Polymers wie auch in der Variation der Additive läßt sich zudem eine schichtabhängige Differenzierung bezüglich der eingesetzten Wirkstoffe als auch bezüglich der Konzentration vornehmen. Diese Adaptionsfähigkeit zeichnet die Polysulfonmatrix als ein universell einsetzbares biostabiles Beschichtungsmaterial zur Verhinderung der Restenose aus.

Ferner ist die Verwendung von thermoplastischen Polysulfonen bevorzugt. Thermoplastische Polysulfone lassen sich unter dem Einfluss von Wärme (Thermo) plastisch (plaste) verformen. In der Regel bestehen thermoplastische Polysulfone aus linearen oder wenig verzweigten Molekülketten. Werden sie erwärmt, lassen sie sich durch Zug strecken. Bei stärkerer Erwärmung können sie vollständig eingeschmolzen und neu geformt werden. Insbesondere ist bevorzugt, wenn diese thermoplastischen Polysulfone sowohl hydrophile als auch hydrophobe Eigenschaften aufweisen. Derartige thermoplastische Polysulfone mit diesen ambivalenten Eigenschaften können gemäß den oben beschriebenen Verfahren durch polymeranaloge Reaktionen, Blockcopolymerisation oder Polymerisation von hydrophilen mit hydrophoben Monomeren hergestellt werden. Die so erhaltenen thermoplastischen Polymere bzw. die damit beschichteten Medizinprodukte zeichnen sich durch mehrfache Sterilisierbarkeit, Heißdampf- und Hydrolysebeständigkeit, hohe Dimensionssabilität, Resistenz gegen aggressive Chemikalien, sowie gute Wärmealterungsbeständigkeit aus.

Ein bevorzugtes thermoplastisches Polysulfon wird aus Bisphenol A und 4,4'-Dichlorphenylsulfon über Polykondensationsreaktionen hergestellt (s. nachfolgende Formel (II)). 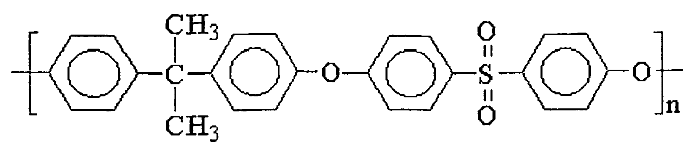 *Poly[oxy-1,4-phenylen-sulfonyl-1,4-phenylen-oxy-(4,4'-isopropylidendiphenylen)*]

Die für die erfindungsgemäße Beschichtung einsetzbaren Polysulfone haben folgende allgemeine Struktur gemäß Formel (I): 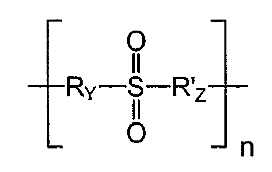 worin
n den Polymerisationsgrad bedeutet, der im Bereich von n = 10 bis n = 10.000, bevorzugt im Bereicht von n = 20 bis n = 3.000, weiter bevorzugt im Bereich von n = 40 bis n = 1.000, weiter bevorzugt im Bereich von n = 60 bis n = 500, weiter bevorzugt im Bereich von n = 80 bis n = 250 und insbesondere bevorzugt im Bereich von n = 100 bis n = 200 liegt.
Ferner ist bevorzugt, wenn n in einem solchen Bereich liegt, so dass sich ein Gewichtsmittel des Polymeren von 60.000 - 120.000 g/mol, bevorzugt von 70.000 bis 99.000 g/mol, weiter bevorzugt von 80.000 - 97.000, noch weiter bevorzugt von 84.000 - 95.000 und insbesondere bevorzugt von 86.000 - 93.000 g/mol ergibt.
Zudem ist bevorzugt, wenn n in einem derartigen Bereich liegt, dass sich für das Zahlenmittel des Polymeren ein Bereicht von 20.000 - 70.000 g/mol, bevorzugt von 30.000 - 65.000 g/mol, weiter bevorzugt von 32.000 - 60.000, noch weiter bevorzugt von 35.000 - 59.000, besonders bevorzugt ein Bereich von 45.000 - 58.000 g/mol ergibt.

y und z sind ganze Zahlen im Bereich von 1 bis 10, und R und R' bedeuten unabhängig voneinander eine Alkylengruppe mit 1 bis 12 Kohlenstoffatomen, eine aromatische Gruppe mit 6 bis 20 Kohlenstoffatomen, eine heteroaromatische Gruppe mit 2 bis 10 Kohlenstoffatomen, eine Cycloalkylengruppe mit 3 bis 15 Kohlenstoffatomen, eine Alkylenarylengruppe mit 6 bis 20 Kohlenstoffatomen, eine Arylenalkylengruppe mit 6 bis 20 Kohlenstoffatomen, eine Alkylenoxygruppe mit 1 bis 12 Kohlenstoffatomen, eine Arylenoxygruppe mit 6 bis 20 Kohlenstoffatomen, eine Heteroarylenoxygruppe mit 6 bis 20 Kohlenstoffatomen, eine Cycloalkylenoxygruppe mit 3 bis 15 Kohlenstoffatomen, eine Alkylenarylenoxygruppe mit 6 bis 20 Kohlenstoffatomen oder eine Arylenalkylenoxygruppe mit 6 bis 20 Kohlenstoffatomen. Die vorgenannten Gruppen können weitere Substituenten tragen, insbesondere solche die weiter unten unter "substituierte" Polysulfone beschrieben sind.

Beispiele für die Gruppen R und R' sind -R¹-, -R²-, -R³-, -R⁴-, -R⁵-, -R⁶-, -R¹-R², -R³-R⁴-, -R⁵-R⁶-, -R¹-R²-R³-, -R⁴-R⁵-R⁶-, -R¹-R²-R³-R⁴-, -R¹-R²-R³-R⁴-R⁵- sowie -R¹-R²-R³-R⁴-R⁵-R⁶-;
wobei R¹, R², R³, R⁴, R⁵ und R⁶ unabhängig voneinander folgende Gruppen bedeuten:
-CH₂-, -C₂H₄-, -CH(OH)-, -CH(SH)-, -CH(NH₂)-, -CH(OCH₃)-, -C(OCH₃)₂-, -CH(SCH₃)-, -C(SCH₃)₂-, -CH(NH(CH₃))-, -C(N(CH₃)₂)-, -CH(OC₂H₅)-, -C(OC₂H₅)₂-, -CHF-, -CHCl-, -CHBr-, -CF₂-, -CCl₂-, -CBr₂-, -CH(COOH)-, -CH(COOCH₃)-, -CH(COOC₂H₅)-, -CH(COCH₃)-, -CH(COC₂H₅)-, -CH(CH₃)-, -C(CH₃)₂-, -CH(C₂H₅)-, -C(C₂H₅)₂-, -CH(CONH₂)-, -CH(CONH(CH₃))-, -CH(CON(CH₃)₂)-,
-C₃H₆-, -C₄H₈-, -C₅H₉-, W₆H₁₀-, cyclo-C₃H₄-, cyclo-C₃H₄-, cyclo-C₄H₆-, cyclo-C₅H₈-, -OCH₂-, -OC₂H₄-, -OC₃H₆-, -OC₄H₈-, -OC₅H₉-, -OC₆H₁₀-, -CH₂O-, -C₂H₄O-, -C₃H₆O-, -C₄H₈O-, -C₅H₉O-, -C₆H₁₀O-, -NHCH₂-, -NHC₂H₄-, -NHC₃H₆-, -NHC₄H₈-, -NHC₅H₉-, -NHC₆H₁₀-, -CH₂NH-, -C₂H₄NH-, -C₃H₆NH-, -C₄H₈NH-, -C₅H₉NH-, -C₆H₁₀NH-, -SCH₂-, -SC₂H₄-, -SC₃H₆-, -SC₄H₈-, -SC₅H₉-, -SC₆H₁₀-, -CH₂S-, -C₂H₄S-, -C₃H₆S-, -C₄H₈S-, -C₅H₉S-, -C₆H₁₀S-,
-C₆H₄-, -C₆H₃(CH₃)-, -C₆H₃(C₂H₅)-, -C₆H₃(OH)-, -C₆H₃(NH₂)-, -C₆H₃(C))-, -C₆H₃(F)-, -C₆H₃(Br)- -C₆H₃(OCH₃)- -C₆H₃(SCH₃)-, -C₆H₃(COCH₃)-, -C₆H₃(COC₂H₅)-, -C₆H₃(COOH)-, -C₆H₃(COOCH₃)-. -C₆H₃(COOC₂H₅)-, -C₆H₃(NH(CH₃))-, -C₆H₃(N(CH₃)₂)-, -C₆H₃(CONH₂)-, -C₆H₃(CONH(CH₃))-, -C₆H₃(CON(CH₃)₂)-,
-OC₆H₄-, -OC₆H₃(CH₃)-, -OC₆H₃(C₂H₅)-, -OC₆H₃(OH)-, -OC₆H₃(NH₂)-, -OC₆H₃(Cl)-, -OC₆H₃(F)-, -OC₆H₃(Br)-, -OC₆H₃(OCH₃)-, -OC₆H₃(SCH₃)-, -OC₆H₃(COCH₃)-, -OC₆H₃(COC₂H₅)-, -OC₆H₃(COOH)-, -OC₆H₃(COOCH₃)-, -OC₆H₃(COOC₂H₅)-, -OC₆H₃(NH(CH₃))-, -OC₆H₃(N(CH₃)₂)-, -OC₆H₃(CONH₂)-, -OC₆H₃(CONH(CH₃))-, -OC₆H₃(CON(CH₃)₂)-,
-C₆H₄O-, -C₆H₃(CH₃)O-, -C₆H₃(C₂H₅)O-, -C₆H₃(OH)O-, -C₆H₃(NH₂)O-, -C₆H₃(Cl)O-, -C₆H₃(F)O-, -C₆H₃(Br)O-, -C₆H₃(OCH₃)O-, -C₆H₃(SCH₃)O-, -C₆H₃(COCH₃)O-, -C₆H₃(COC₂H₅)O-, -C₆H₃(COOH)O-, -C₆H₃(COOCH₃)O-, -C₆H₃(COOC₂H₅)O-, -C₆H₃(NH(CH₃))O-, -C₆H₃(N(CH₃)₂)O-, -C₆H₃(CONH₂)O-, -C₆H₃(CONH(CH₃))O-, -C₆H₃(CON(CH₃)₂)O-,
-SC₆H₄-, -SC₆H₃(CH₃)-, -SC₆H₃(C₂H₅)-, -SC₆H₃(OH)-, -SC₆H₃(NH₂)-, -SC₆H₃(Cl)-, -SC₆H₃(F)-, -SC₆H₃(Br)-, -SC₆H₃(OCH₃)-, -SC₆H₃(SCH₃)-, -SC₆H₃(COCH₃)-, -SC₆H₃(COC₂H₅)-, -SC₆H₃(COOH)-, -SC₆H₃(COOCH₃)-, -SC₆H₃(COOC₂H₅)-, -SC₆H₃(NH(CH₃))-, -SC₆H₃(N(CH₃)₂)-, -SC₆H₃(CONH₂)-, -SC₆H₃(CONH(CH₃))-, -SC₆H₃(CON(CH₃)₂)-,
-C₆H₄S-, -CeH₃(CH₃)S-, -C₆H₃(C₂H,₅)S-, -C₆H₃(OH)S-, -C₆H₃(NH₂)S-, -C₆H₃(Cl)S-, -C₆H₃(F)S-, -C₆H₃(Br)S-, -C₆H₃(OCH₃)S-, -C₆H₃(SCH₃)S-, -C₆H₃(COCH₃)S-, -C₆H₃(COC₂H₅)S-, -C₆H₃(COOH)S-, -C₆H₃(COOCH₃)S-, -C₆H₃(COOC₂H₅)S-, -C₆H₃(NH(CH₃))S-, -C₆H₃(N(CH₃)₂)S-, -C₆H₃(CONH₂)S-, -C₆H₃(CONH(CH₃))S-, -C₆H₃(CON(CH₃)₂)S-,
-NH-C₆H₄-, -NH-C₆H₃(CH₃)-, -NH-C₆H₃(C₂H₅)-, -NH-C₆H₃(OH)-, -NH-C₆H₃(NH₂)-, -NH-C₆H₃(Cl)-, -NH-C₆H₃(F)-, -NH-C₆H₃(Br)-, -NH-C₆H₃(OCH₃)-, -NH-C₆H₃(SCH₃)-, -NH-C₆H₃(COCH₃)-, -NH-C₆H₃(COC₂H₅)-, -NH-C₆H₃(COOH)-, -NH-C₆H₃(COOCH₃)-, -NH-C₆H₃(COOC₂H₅)-, -NH-C₆H₃(NH(CH₃))-, -NH-C₆H₃(N(CH₃)₂)-, -NH-C₆H₃(CONH₂)-, -NH-C₆H₃(CONH(CH₃))-, -NH-C₆H₃(CON(CH₃)₂)-,
-C₆H₄-NH-, -C₆H₃(CH₃)-NH-, -C₆H₃(C₂H₅)-NH-, -C₆H₃(OH)-NH-, -C₆H₃(NH₂)-NH-, -C₆H₃(Cl)-NH-, -C₆H₃(F)-NH-, -C₆H₃(Br)-NH-, -C₆H₃(OCH₃)-NH-, -C₆H₃(SCH₃)-NH-, -C₆H₃(COCH₃)-NH-, -C₆H₃(COC₂H₅)-NH-, -C₆H₃(COOH)-NH-, -C₆H₃(COOCH₃)-NH-, -C₆H₃(COOC₂H₅)-NH-, -C₆H₃(NH(CH₃))-NH-, -C₆H₃(N(CH₃)₂)-NH-, -C₆H₃(CONH₂)-NH-, -C₆H₃(CONH(CH₃))-NH-, -C₆H₃(CON(CH₃)₂)-NH-.

Besonders bevorzugt werden Polysulfone sowie ihre Mischungen, worin die Gruppen -R¹-, -R²-, -R³-, -R¹-R²-, -R¹-R²-R³- unabhängig voneinander folgende Gruppen bedeuten: -C₆H₄O-, -C(CH₃)₂-, -C₆H₄-, -C₆H₄SO₂-, -SO₂C₆H₄-, -OC₆H₄-, -C₆H₄O-C(CH₃)₂-C₆H₄-.

R und R' können ferner unabhängig voneinander bevorzugt einen der an die Sulfongruppe in den Formel (II) bis (XV) gebundenen Rest darstellen.

Erfindungsgemäß wird das Polysulfon bzw. werden die Polysulfone für die biostabile Schicht oder die biostabilen Schichten aus der Gruppe ausgewählt umfassend: Polyethersulfon, substituiertes Polyethersulfon, Polyphenylsulfon, substituiertes Polyphenylsulfon, Polysulfonblockcopolymere, perfluorierte Polysulfonblockcopolymere, semifluorierte Polysulfonblockcopolymere, substituierte Polysulfonblockcopolymere und/oder Mischungen der vorgenannten Polymere. Unter dem Begriff "substituierte" Polysulfone werden Polysulfone verstanden, welche funktionelle Gruppen aufweisen. Insbesondere können die Methyleneinheiten einen oder zwei Substituenten und die Phenyleneinheiten einen, zwei, drei oder vier Substituenten aufweisen. Beispiele für diese Substituenten (auch bezeichnet als X, X', X", X"') sind: -OH, -OCH₃, -OC₂H₅, -SH, -SCH₃, -SC₂H₅, -NO₂, -F, -Cl, -Br, -I, -N₃, -CN, -OCN, -NCO, -SCN, -NCS, -CHO, -COCH₃, -COC₂H₅, -COOH, -COCN, -COOCH₃, -COOC₂H₅, -CONH₂, -CONHCH₃, -CONHC₂H₅, -CON(CH₃)₂, -CON(C₂H₅)₂, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -OCF₃, -O-COOCH₃, -O-COOC₂H₅, -NH-CO-NH₂, -NH-CS-NH₂, -NH-C(=NH)=NH₂, -O-CO-NH₂, -NH-CO-OCH₃, -NH-CO-OC₂H₅, -CH₂F -CHF₂, -CF₃, -CH₂Cl -CHCl₂, -CCl₃, -CH₂Br -CHBr₂, -CBr₃, -CH₂l -CHl₂, -Cl₃, -CH₃, -C₂H₅, -C₃H₇, -CH(CH₃)₂, -C₄Hg, -CH₂-CH(CH₃)₂, -CH₂-COOH, -CH(CH₃)-C₂H₅, -C(CH₃)₃, -H. Weitere bevorzugte Substituenten oder funktionelle Gruppen sind -CH₂-X und -C₂H₄-X.

Die nachfolgenden allgemeinen Strukturformeln repräsentieren bevorzugte Wiederholungseinheiten für Polysulfone. Bevorzugt bestehen die Polymere nur aus diesen Wiederholungseinheiten. Es ist aber auch möglich, dass in einem Polymeren neben den gezeigten Wiederholungseinheiten noch andere Wiederholungseinheiten oder Blöcke vorhanden sind. Bevorzugt sind: 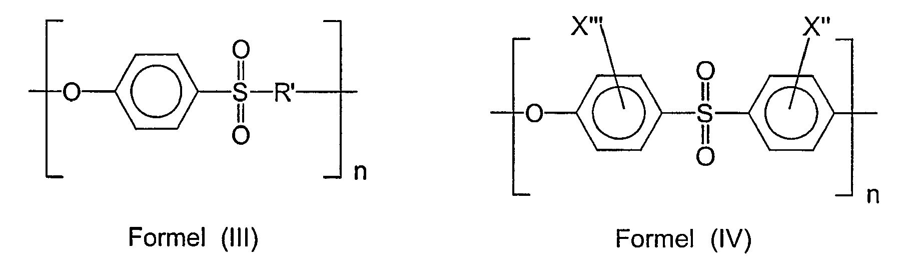 X, X', n und R' haben unabhängig voneinander die oben erwähnte Bedeutung. 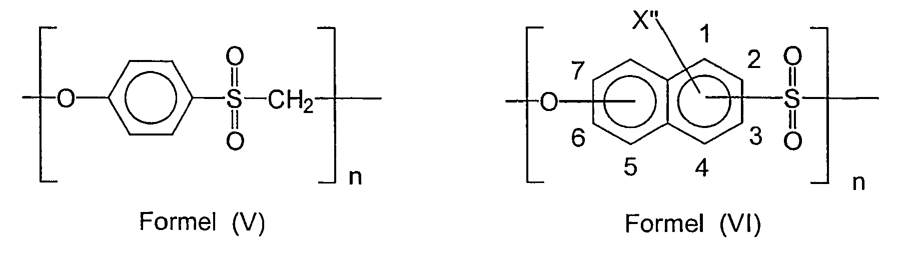 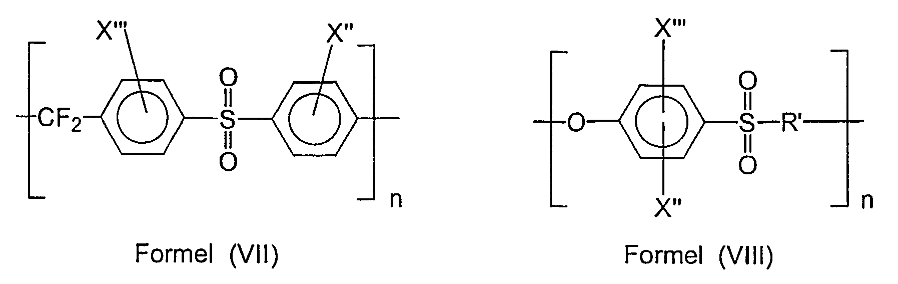 X, X', n und R' haben unabhängig voneinander die oben erwähnte Bedeutung.

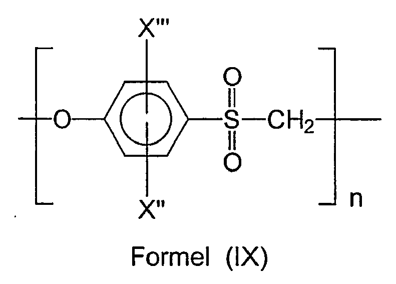

Ferner sind Polysulfone der folgenden allgemeinen Formel (X) bevorzugt 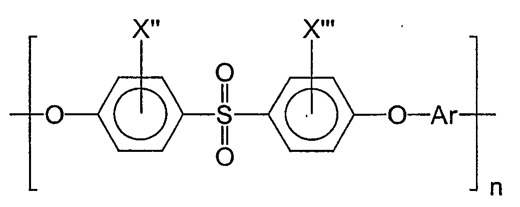 worin Ar bedeutet 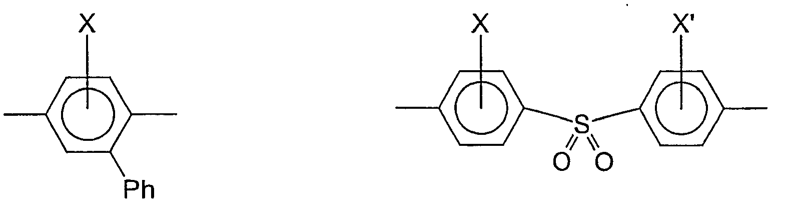 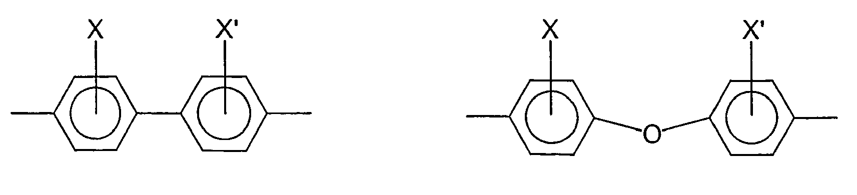 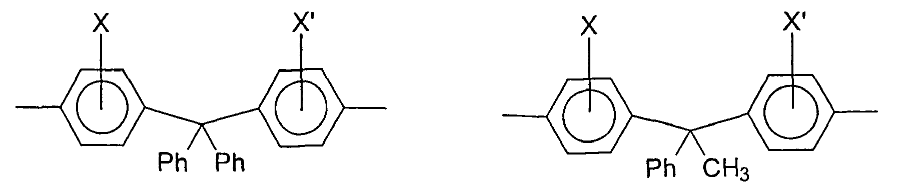 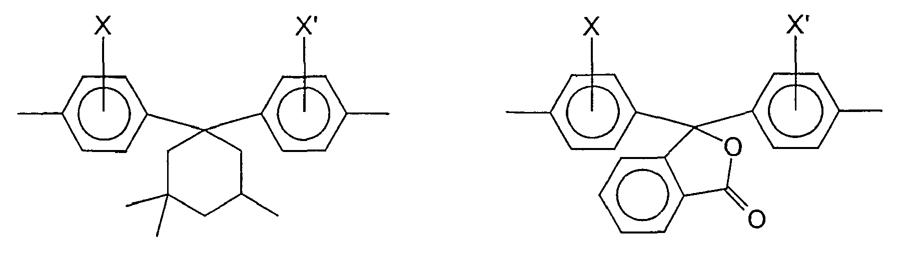 X, X' und n haben unabhängig voneinander die oben erwähnte Bedeutung.

Folgende Wiederholungseinheiten sind des weiteren bevorzugt 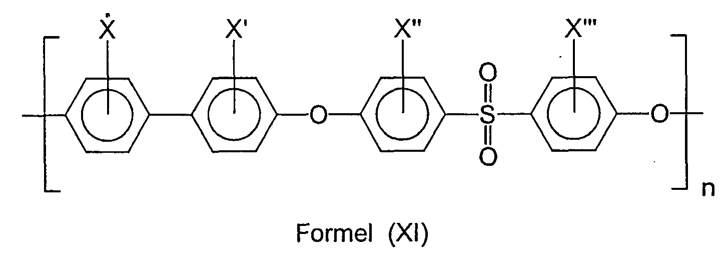 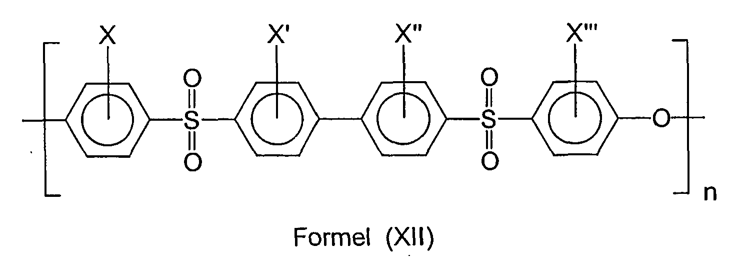 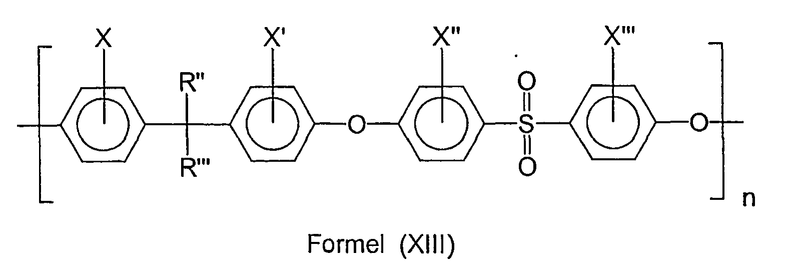 X, X', X", X'" und n haben unabhängig voneinander die oben erwähnte Bedeutung. R" und R"' können unabhängig voneinander für einen Substituenten stehen, wie er für X oder X' definiert ist oder können unabhängig voneinander einen Rest -R¹-H oder -R²-H bedeuten.

Eine weitere bevorzugte Wiederholungseinheit weist einen cyclischen Substituenten zwischen zwei aromatischen Ringen auf, wie beispielsweise Formel (XIV) oder (XV): 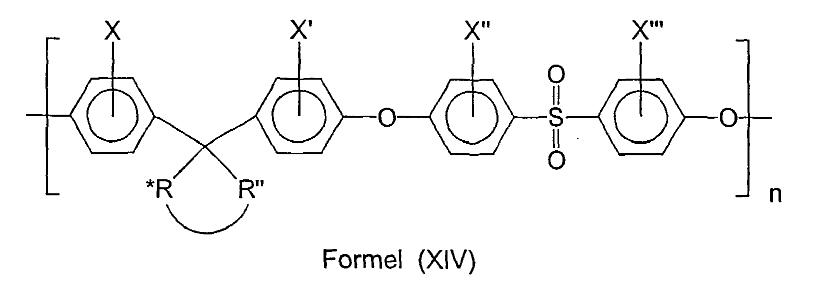 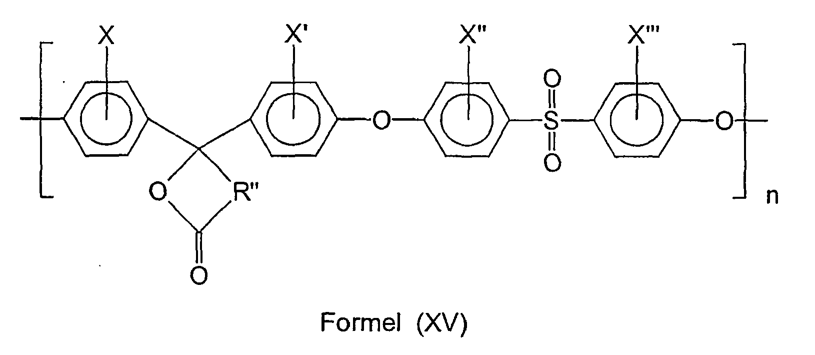 R" bedeutet bevorzugt -CH₂-, -OCH₂-, -CH₂O-, -O-, -C₂H₄-, -C₃H₆-, -CH(OH)-. Die Gruppe -*R-R"- bedeutet bevorzugt einen cyclischen Ester, Amid, Carbonat, Harnstoff oder Urethan wie beispielsweise: -O-CO-O-, -O-CO-O-CH₂-, -O-CO-O-C₂H₄-, -CH₂-O-CO-O-CH₂-, -C₂H₄-, -C₃H₆-, -C₄H₈-, -C₅H₁₀-, -C₆H₁₂-, -O-CO-NH-, -NH-CO-NH-, -O-CO-NH-CH₂-, -O-CO-NH-C₂H₄-, -NH-CO-NH-CH₂-, -NH-CO-NH-C₂H₄-, -NH-CO-O-CH₂-, -NH-CO-O-C₂H₄-, -CH₂-O-CO-NH-CH₂-, -C₂H₄-SO₂-, -C₃H₆-SO₂-, -C₄H₈-SO₂-, -C₂H₄-SO₂-CH₂-, -C₂H₄-SO₂-C₂H₄-, -C₂H₄-O-, -C₃H₆-O-, -C₄H₈-O-, -C₂H,-O-CH₂-, -C₂H,-O-C₂H₄-, -C₂H₄-CO-, -C₃H₆-CO-, -C₄H₈-CO-, -C₂H₄-CO-CH₂-, -C₂H₄-CO-C₂H₄-, -O-CO-CH₂-, -O-CO-C₂H₄-, -O-CO-C₂H₂-, -CH₂-O-CO-CH₂-, oder cyclische Ester enthaltend einen aromatischen Ring.

Im folgenden werden polymeranaloge Reaktionen beschrieben, welche dem Fachmann bekannt sind und zur Modifizierung der Polysulfone dienen

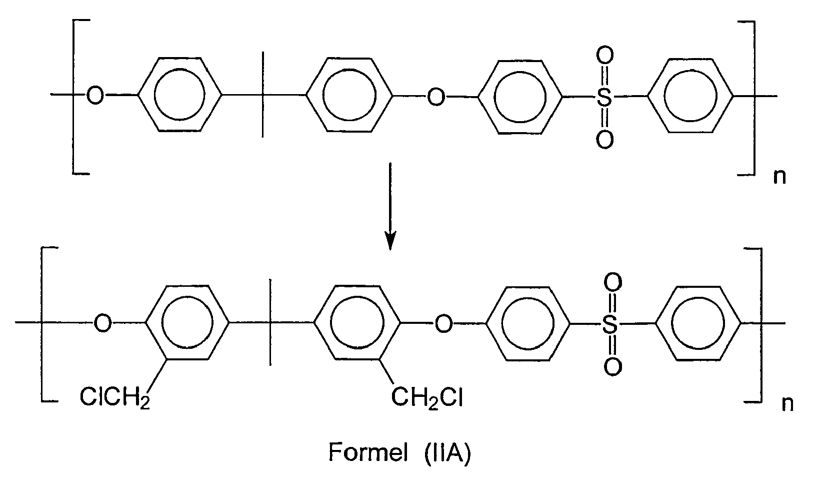

Chlormethylengruppen als Reste X und X' lassen sich unter Verwendung von Formaldehyd, CISMe₃ und einem Katalysator wie SnCl₄ einführen, welche danach weiter substituiert werden können. Durch diese Reaktion lassen sich beispielsweise Hydroxygruppen, Aminogruppen, Carboxylatgruppen, Ether oder Alkylreste durch eine nucleophile Substitution einführen, welche über eine Methylengruppe an den Aromaten gebunden sind. Eine Umsetzung mit Alkoholaten wie beispielsweise einem Phenolat, Benzylat, Methanolat, Ethanolat, Propanolat oder Isopropanolat führt zu einem Polymer, bei dem an mehr als 75% der Chlormethylengruppen eine Substitution stattgefunden hat. Man erhält folgendes Polysulfon mit lipophilen Seitengruppen: 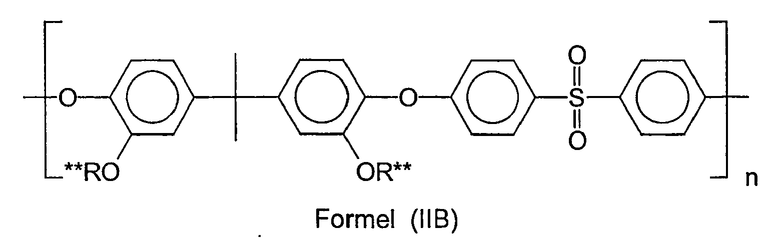 worin
R** beispielsweise einen Alkylrest oder Arylrest bedeutet.

Die Reste X" und X"' lassen sich, soweit in den Monomeren noch nicht vorhanden, am Polymeren durch folgende Reaktion einführen:

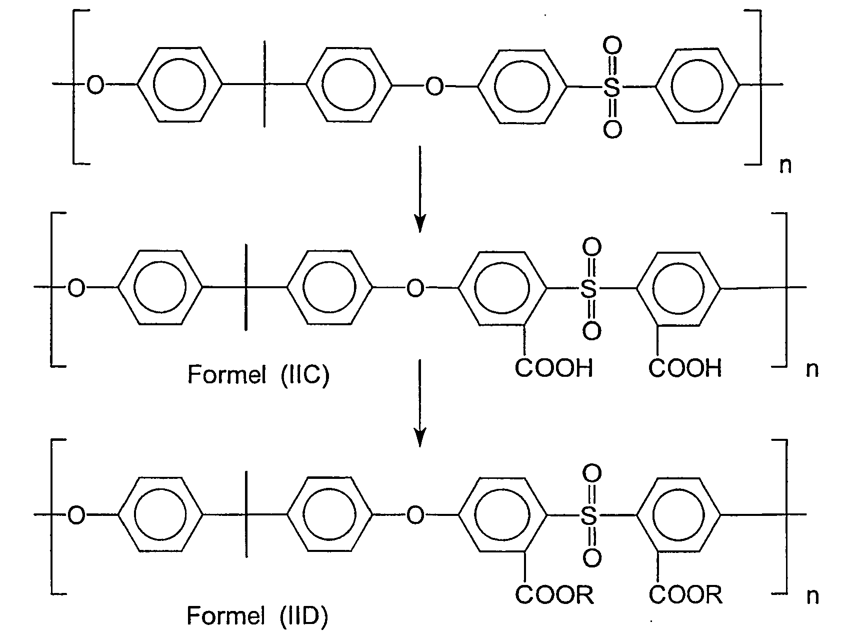

Neben einer Estergruppe können diverse andere Substituenten eingeführt werden, indem zuerst mit einer starken Base, z.B. n-BuLi oder tert-BuLi eine einfache oder zweifache Deprotonierung durchgeführt wird und danach ein Elektrophil zugesetzt wird. Im obigen Beispielsfall wurde zur Einführung der Estergruppe Kohlendioxid zugesetzt und die erhaltene Carbonsäuregruppe in einem weiteren Schritt verestert.

Eine erfindungsgemäße Kombination aus einem Polysulfon mit lipophilen Resten und einem Polysulfon mit lipophoben Resten wird beispielsweise durch die Verwendung von Polysulfon gemäß Formel (IIB) zusammen mit Polysulfon gemäß Formel (IIC) erreicht. Die Mengenverhältnisse von beiden Polysulfonen zueinander können von 98% : 2% bis 2% zu 98% liegen. Bevorzugte Bereiche sind 10% zu 90%, 15% zu 85%, 22% zu 78% und 27% zu 73%, 36% zu 64%, 43% zu 57% und 50% zu 50%. Diese Prozentangaben gelten für beliebige Kombinationen von hydrophilen und hydrophoben Polysulfonen und sind nicht auf die vorgenannte Mischung beschränkt.

Ein Beispiel für ein Polysulfon mit hydrophilen und hydrophoben Resten in einem Molekül erhält man beispielsweise, indem das Polysulfon gemäß Formel (IIC) nur unvollständig verestert wird und somit hydrophile Carboxylatgruppen und hydrophobe Estergruppen in einem Molekül vorliegen. Das Molverhältnis (Anzahl) an Carboxylatgruppen zu Estergruppen kann 5% zu 95% bis 95% zu 5% betragen. Diese Prozentangaben gelten für beliebige Kombinationen von hydrophilen und hydrophoben Gruppen und sind nicht auf die vorgenannten beschränkt.

Es wird vermutet, dass durch diese erfindungsgemäße Kombination von hydrophilen Gruppen bzw. Polymeren mit hydrophoben Gruppen bzw. Polymeren sich amorphe Polymerschichten auf dem Medizinprodukt ausbilden. Es ist sehr wichtig, dass die Polymerschichten aus Polysulfon nicht kristallin oder überwiegend kristallin sein dürfen, da Kristallinität zu starren Schichten führt, welche einreißen und sich ablösen. Flexible als Barriereschicht dienende Polysulfonbeschichtungen lassen sich nur mit amorphen oder überwiegend amorphen Polysulfonschichten erreichen.

Natürlich ist es auch möglich, bereits entsprechend substituierte Monomere einzusetzen, um nach erfolgter Polymerisation das gewünschte Substitutionsmuster zu erhalten. Die entsprechenden Polymere ergeben sich dann in bekannter Weise gemäß folgenden Reaktionsschema: 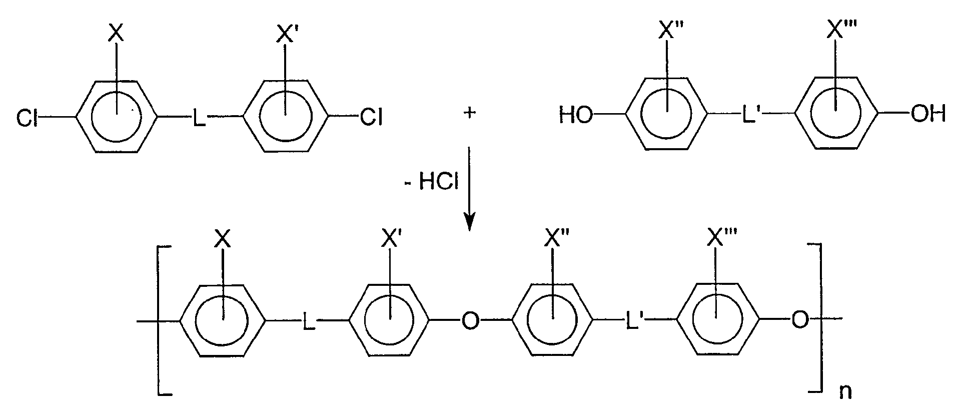 worin
L und L' unabhängig voneinander beispielsweise für folgende Gruppen stehen: -SO₂-, -C(CH₃)₂-, -C(Ph)₂- oder -O-. L und L' können somit die Bedeutungen der entsprechenden Gruppen in den Formeln (I) bis (XV) haben. Dem Fachmann sind derartige nucleophile Substitutionsreaktionen bekannt, welche durch das obige Schema beispielhaft verdeutlicht werden.

Wie bereits erwähnt, ist insbesondere bevorzugt, wenn die Polymere hydrophile und hydrophobe Eigenschaften aufweisen, zum einen innerhalb eines Polymers und zum anderen durch Verwendung mindestens eines hydrophilen Polymers in Kombination mit mindestens einem hydrophoben Polymeren. Somit ist bevorzugt, wenn es sich beispielsweise bei X und X' um hydrophile Substituenten und bei X" und X"' um hydrophobe Substituenten handelt oder umgekehrt.

Als hydrophile Substituenten kommen in Frage: -OH, -CHO, -COOH, -COO⁻,-CONH₂, -NH₂, -N⁺(CH₃)₄, -NHCH₃, -SO₃H, -SO₃⁻, -NH-CO-NH₂, -NH-CS-NH₂, -NH-C(=NH)-NH₂, -O-CO-NH₂ und insbesondere protonierte Aminogruppen.

Als hydrophobe Substituenten kommen in Frage: -H, -OCH₃, -OC₂H₅, -SCH₃, -SC₂H₅, -NO₂, -F, -Cl, -Br, -I, -N₃, -CN, -OCN, -NCO, -SCN, -NCS, -COCH₃, -COC₂H₅, -COCN, -COOCH₃, -COOC₂H₅, -CONHC₂H₅, -CON(CH₃)₂, -CON(C₂H₅)₂, -NHC₂H₅, -N(CH₃)₂, -N(C₂H₅)₂, -SOCH₃, -SOC₂Hs, -SO₂CH₃, -SO₂C₂H₅, -SO₃CH₃, -SO₃C₂H₅, -OCF₃, -O-COOCH₃, -O-COOC₂H₅, -NH-CO-OCH₃, -NH-CO-OC₂H₅, -CH₂F -CHF₂, -CF₃, -CH₂Cl -CHCl₂, -CCl₃, -CH₂Br -CHBr₂, -CBr₃, -CH₂l -CHl₂, -Cl₃, -CH₃, -C₂H₅, -C₃H₇, -CH(CH₃)₂, -C₄H₉, -CH₂-CH(CH₃)₂, -CH₂-COOH, -CH(CH₃)-C₂H₅, -C(CH₃)₃.

Des weiteren sind cyclische Polysulfone bevorzugt, welche beispielsweise eine Struktur wie in Formel (XVI) gezeigt aufweisen:

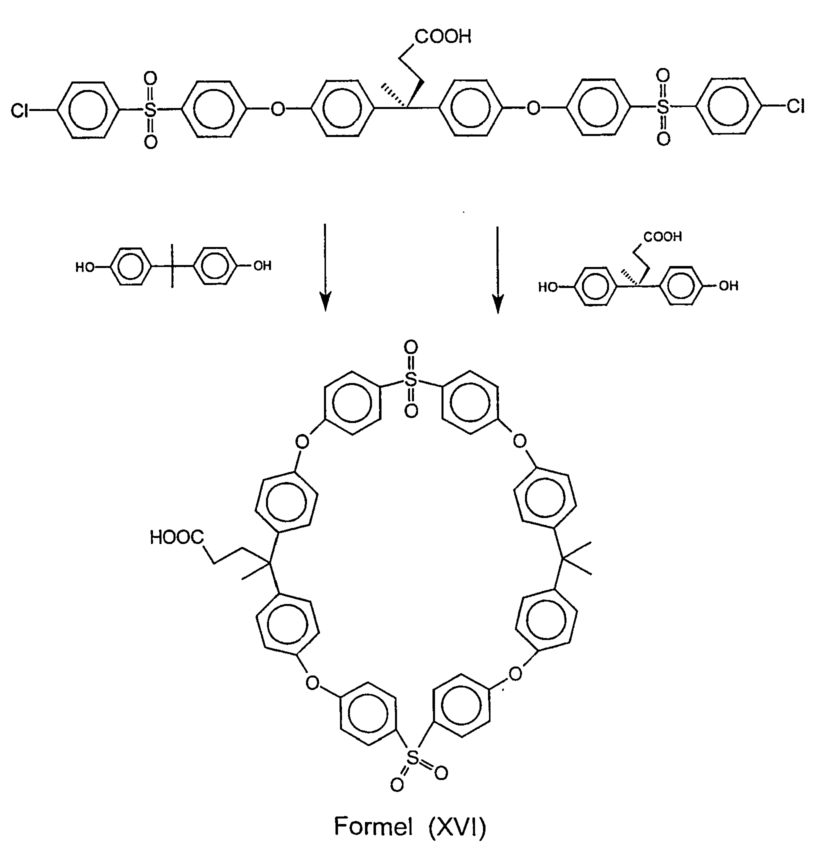

Die Carboxyethylengruppe ist für die obige Beispielsreaktion nicht essentiell. Anstelle der Carboxyethylen- und der Methylsubstituenten können beliebige andere Substituten oder auch Wasserstoff vorhanden sein.

Polysulfone zeichnen sich durch ihre hohe Resistenz gegen aggressive Chemikalien aus, sie sind hydrolysebeständig und wärmebeständig und haben sehr gute mechanische und tribologische (keinen Oberflächenabrieb) Eigenschaften. Als Material für den Einsatz im lebenden Organismus lassen sich als weitere besondere Eigenschaften die hohe Dimensionsstabilität und die mehrfache Sterilisierbarkeit hervorheben. Polysulfone sind als medizinische Polymere schon seit langer Zeit im Einsatz. Die hauptsächliche Verwendung konzentriert sich auf Hohlfasern z.B. in Blutdialysatoren, wo sich die Polysulfonfaser der Firma Fresenius aufgrund ihrer guten Hämokopatibilität und Membranbildungseigenschaften auf dem weltweiten Markt als führend durchgesetzt hat. Die Problematik der Dialyse besteht dabei vor allem aus der Notwendigkeit, dass während der Blutwäsche ein Antikoagulanz, in der Regel Heparin, zugegeben werden muss, dessen Nebenwirkungen nach ein paar Jahren Überhand nehmen. Während einer fünfstündigen Behandlung fließen ca. 75 Liter Blut - dies entspricht ca. der 15-fachen vorhandenen Blutmenge des Patienten - durch den Dialysator. Damit wird deutlich, dass an die Membran eine sehr hohe Hämokompatibilitätsanforderung gestellt ist.
Ein weiterer großer Bereich ist die Anwendung von Polysulfon-Kapillaren in der Augenheilkunde und in Form von Flachmembranen in diversen medizintechnischen Hilfsmitteln.

Bevorzugt ist, wenn dem für die biostabile Schicht verwendeten Polysulfon mindestens ein hydrophiles Polymer zugesetzt wird. Dabei kann in der jeweiligen Polysulfonschicht das Verhältnis von Polysulfon zu hydrophilem Polymer 50 Gew.-% zu 50 Gew.-% bis 99,999 Gew.-% zu 0,001 Gew.-% betragen.

Als hydrophile Polymere eignen sich Polyvinylpyrrolidon, Glycerin, Polyethylenglykol, Polypropylenglycol, Polyvinylalkohol, Polyhydroxyethyl-methacrylate, Polyacrylamid, Polyvalerolactone, Poly-ε-Decalactone, Polylactonsäure, Polyglycolsäure, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Fibrin, Polycyanoacrylate, Polycaprolactondimethylacrylate, Poly-b-Maleinsäure, Polycaprolactonbutyl-acrylate, Multiblockpolymere wie z.B. aus Oligocaprolactondiole und Oligodioxanondiole, Polyetherestermultiblockpolymere wie z.B. PEG und Poly(butylenterephtalat), Polypivotolactone, Polyglycolsäuretrimethylcarbonate, Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Chitosan und seine Derivate, Chondroitinsulfat, Dextrane, b-Cyclodextrine, Copolymere mit PEG und Polypropylenglycol, Gummi arabicum, Guar, Gelatine, Collagen, Collagen-N-Hydroxysuccinimid, Lipide, Phospholipide, Modifikationen und Copolymere und/oder Mischungen der vorgenannten Substanzen eingesetzt, wobei Polyvinylpyrrolidon, Polyethylenglycol und Glycerin bevorzugt eingesetzt werden.

Beispielsweise wird zur Steigerung der Viskosität bei der Produktion der Polysulfonlösung Polyvinylpyrrolidon (PVP) zugesetzt, das während der Hohlfaserherstellung im Fällmittel löslich ist und so wieder entfernt wird. Die fertige poröse Hohlfaser enthält durchschnittlich noch einen prozentualen Anteil von 1-2 %PVP. Der Zusatz von Polyvinylpyrrolidon ist nicht nur der Viskosität während der Produktion zuträglich, d.h. wirkt viskositätserhöhend, sondern gleichfalls ein die Porengröße des Polysulfons mitbestimmender Faktor und damit für die Permeabilitätseigenschaften des Endproduktes maßgeblich, denn diese ist von der Porengröße und Teilchengröße abhängig. Somit läßt sich über die Menge und das Molekulargewicht des zugefügten Polyvinylpyrrolidons die Porengröße und damit die Permeabilität des produzierten Polysulfons regulieren.
Die biokompatiblen und guten mechanischen Eigenschaften von Polysulfon und die Möglichkeit zur Regulation der Porengröße durch den Zusatz von Polyvinylpyrrolidon und/oder einem anderen hydrophilen Polymeren oder/und Wasser (Essigsäureethylester) macht dieses Polymer zum idealen Träger für alle Arzneimittel. die zur gezielten lokalen Applikation, wie z.B. in der Kardiologie zur Verhinderung von Gefäßwiederverschlüssen, einsetzbar sind.
Gleichzeitig sorgt der eingeschlossene Stickstoff für die Haltbarkeit des Wirkstoffes.

Die bevorzugte Menge des zugesetzten Polymeren liegt im Bereich von 0-50 Gew.-%, weiter bevorzugt sind 1-20 Gew.-%, besonders bevorzugt werden 2-10% Gew.-%.
Die zugesetzte Menge richtet sich im wesentlichen nach der gewünschten Elutionsgeschwindigkeit des eingesetzten Wirkstoffes.

Die erfindungsgemäßen Medizinprodukte besitzen eine Oberfläche, welche aus beliebigem Material bestehen kann. Diese Oberfläche ist bevorzugt nicht hemokompatibel. Ferner ist diese Oberfläche bevorzugt nicht beschichtet, insbesondere nicht mit Polymeren und/oder organischen Makromolekülen.

An diese Oberfläche kann die biostabile Schicht aus Polysulfon adhäsiv oder kovalent als auch teilweise adhäsiv und teilweise kovalent gebunden werden. Bevorzugt ist die kovalente Anbindung. Die Schicht aus Polysulfon bedeckt die Oberfläche des Medizinproduktes zumindest teilweise, bevorzugt aber vollständig. Handelt es sich bei dem Medizinprodukt um einen Stent, so ist zumindest die dem Blut ausgesetzte Oberfläche mit dem Polysulfon beschichtet.

Auf diese erste biostabile Schicht aus Polysulfon und/oder in diese erste Schicht aus Polysulfon wird mindestens eine, mindestens einen antiproliferativen, antiinflammatorischen, antiphlogistischen und/oder antithrombotischen Wirkstoff enthaltende Schicht aufgebracht und/oder eingebracht. Die mindestens eine Schicht enthaltend mindestens einen antiproliferativen, antiinflammatorischen, antiphlogistischen und/oder antithrombotischen Wirkstoff kann vollständig aus einem oder mehreren Wirkstoffen bestehen oder eine weitere Schicht aus biostabilem Polysulfon sein, worin sich der Wirkstoff oder die Wirkstoffe befinden oder, kann eine hemokompatible Schicht sein, worin sich der Wirkstoff oder die Wirkstoffe befinden. Während hydrophobe Wirkstoffe sich in und/oder auf und/oder unter eine biostabile Schicht aufbringen lassen, werden hydrophile Wirkstoffe bevorzugt auf und/oder unter einer biostabilen Schicht aufgebracht.

Die erfindungsgemäßen Medizinprodukte können somit Oberflächen aufweisen, welche mit einer, zwei, drei oder mehr Schichten beschichtet sind, wobei eins, zwei oder drei Schichten bevorzugt und insbesondere zwei Schichten bevorzugt sind.

Der oder die antiproliferativen, antiinflammatorischen, antiphlogistischen und/oder antithrombotischen Wirkstoffe können adhäsiv oder kovalent oder zum Teil adhäsiv und zum Teil kovalent an die jeweilige Schicht gebunden werden, wobei die adhäsive Bindung bevorzugt ist.

Weist die Oberflächenbeschichtung mehrere biostabile Schichten aus Polysulfon und/oder hemokompatible Schichten und/oder Wirkstoffschichten auf, so können diese Schichten jeweils aus unterschiedlichen Polysulfonen mit unterschiedlichen hydrophilen Polymeren und unterschiedlichen Mengen an hydrophilen Polymeren sowie unterschieden hemokompatiblen Verbindungen oder unterschiedlichen Wirkstoffen bestehen.

Ferner ist bevorzugt, wenn das hemokompatible Medizinprodukt eine Oberfläche aufweist, welche eine hemokompatible Schicht umfasst, welche auf oder in die unterste erste biostabile Schicht aus Polysulfon aufgebraucht und/oder eingebracht wird. Diese hemokompatible Schicht kann ferner auch eine zweite oder dritte Schicht bilden, welche direkt oder indirekt auf der untersten biostabilen Schicht und/oder auf oder unter einer Wirkstoffschicht oder einer zweiten biostabilen Polysulfonschicht liegt: Zudem ist bevorzugt, wenn die hemokompatible Schicht die unterste Schicht bildet und sich darauf eine Wirkstoffschicht wiederum überdeckt von einer biostabilen Schicht aus Polysulfon befindet oder direkt auf die unterste hemokompatible Schicht eine biostabile Schicht aus Polysulfon mit Wirkstoff oder Wirkstoffkombination aufgebracht wird.

Diese hemokompatible Schicht besteht bevorzugt aus vollständig desulfatiertem und N-reacetyliertem Heparin, desulfatiertem und N-reacetyliertem Heparin, N-carboxymethyliertem, partiell N-acetyliertem Chitosan und/oder aus Mischungen dieser Substanzen. Die hemokompatible Schicht kann neben den vorgenannten Substanzen auch noch weitere hemokompatible organische Substanzen umfassen, besteht bevorzugt aber nur aus den vorgenannten Substanzen.

Bevorzugt ist bei den erfindungsgemäßen hemokompatiblen Medizinprodukten, wenn eine einzige hemokompatible Schicht vorhanden ist. Ferner ist bevorzugt, wenn diese eine hemokompatible Schicht die äußere oder die unterste Schicht bildet.

Ferner ist bevorzugt, dass eine Schicht die darunterliegende Oberfläche oder die darunterliegende Schicht vollständig überdeckt, wobei aber auch eine nur teilweise Überdeckung möglich ist.

Ferner ist insbesondere bevorzugt, wenn es sich bei dem erfindungsgemäßen Medizinprodukt um einen Stent handelt. Dieser Stent kann aus beliebigem Material und Materialmischungen bestehen. Bevorzugt sind Metalle, Metalllegierungen und Kunststoffe wie beispielsweise medizinischer Edelstahl, Titan, Chrom, Vanadium, Wolfram, Molybdän, Gold und Nitinol. Vorzugsweise ist der Stent unbeschichtet und/oder nicht oder nur bedingt hemokompatibel. Insbesondere weist der Stent keine Beschichtung aus organischem Material auf. Medizinische Drähte können als Medizinprodukte ausgeschlossen werden.

Diese erfindungsgemäßen Stents sind bevorzugt mit mindestens einer biokompatiblen vollständig oder unvollständig den Stent überdeckenden Schicht aus biostabilem Polysulfon mit oder ohne einen definierten Anteil eines hydrophilen Polymeren und mit mindestens einem antiproliferativen, entzündungshemmenden, antiphlogistischen und/oder antithrombotischen Wirkstoff versehen. Dabei kann der Wirkstoff in der Matrix und/oder die Matrix als zweite Schicht bedeckend vorliegen. Dabei wird als zweite Schicht diejenige Schicht bezeichnet, welche auf die erste Schicht aufgebracht wird, usw.
Eine weitere bevorzugte Ausführungsform der erfindungsgemäßen Stents weist eine Beschichtung auf, welche aus mindestens zwei Schichten Polysulfon besteht.
Gemäß dieser Zweischichtausführung besteht die erste Schicht aus einer Schicht, welche im wesentlichen vollständig durch eine weitere biostabile Schicht gleicher oder unterschiedlicher Porengröße überzogen ist. Eine oder beide Schichten enthält mindestens einen antiproliferativen, entzündungshemmenden, antiphlogistischen und/oder antithrombotischen Wirkstoff. Ebenso werden auch Wirkstoff-Kombinationen eingesetzt, die sich in ihrer Wirkung gegenseitig unterstützen und/oder ergänzen.
Es besteht ab dieser Zweischichtausführung die Möglichkeit verschiedenartige Wirkstoffe voneinander getrennt in jeweils der für den jeweiligen Wirkstoff geeigneten Schicht unterzubringen, so das sich beispielsweise ein hydrophober Wirkstoff in der einen hydrophileren Schicht aufhält und eine andere Elutionskinetik aufweist als der ein anderer hydrophober Wirkstoff, der in der hydrophoberen Polymerschicht vorliegt oder umgekehrt. Dies bietet ein weites Feld der Möglichkeiten die Verfügbarkeit der Wirkstoffe in eine bestimmte sinnvolle Abfolge zu bringen als auch die Elutionszeit und Konzentration zu steuern.

Eine weitere bevorzugte Ausführungsform der erfindungsgemäßen Stents weist eine Beschichtung auf, welche aus mindestens drei Schichten besteht. Gemäß dieser Dreischichtausführung besteht die erste Schicht aus einer Schicht, welche im wesentlichen vollständig durch eine weitere zweite Schicht aus reinem Wirkstoff oder Wirkstoffkombinationen vollständig bzw. unvollständig überzogen ist, die wiederum von einer dritten biostabilen Polysulfonschicht gleicher oder unterschiedlicher Porengröße bedeckt wird. Die Polysulfonschichten enthalten entweder keinen Wirkstoff oder eine bzw. beide stellen Matrices für mindestens einen antiproliferativen, antiphlogistischen und/oder antithrombotischen Wirkstoff dar.

Ebenso werden auch Wirkstoff-Kombinationen eingesetzt, die sich in ihrer Wirkung gegenseitig unterstützen und/oder ergänzen.
Diese Ausführungsform ist vor allem für die Anwendung hydrophiler Wirkstoffe oder Wirkstoffkombinationen in Form einer reinen Wirkstoffschicht geeignet. Die darüberliegende biostabile Polymerschicht mit einem definierten Gehalt an hydrophilem Polymer dient zur kontrollierten Elution des Wirkstoffes. Wirkstoffkombinationen mit mindestens einem hydrophilen Wirkstoff ergeben unterschiedliche Elutionskinetika.

Als Topcoat läßt sich ebenso das hydrophile Polymer nutzen, das dem auch darunterliegenden Polysulfon zugemischt sein kann.

Die biokompatible Beschichtung eines Stents sorgt für die notwendige Blutverträglichkeit und der Wirkstoff (oder Wirkstoffkombination), der über der Gesamtoberfläche des Stents gleichmäßig verteilt ist, bewirkt, dass der Bewuchs der Stentoberfläche mit Zellen, insbesondere glatten Muskel- und Endothelzellen, in einer kontrollierten Weise abläuft. Somit findet keine rasche Besiedelung und Überwucherung der Stentoberfläche mit Zellen statt, die zu einer Restenose führen könnte, allerdings der Bewuchs der Stentoberfläche mit Zellen aber auch nicht durch eine hohe Medikamentenkonzentration vollkommen unterbunden wird, was die Gefahr einer Thrombose mit sich bringt.

Somit gewährleistet der Einsatz von Polysulfon, dass der Wirkstoff oder die Wirkstoffkombination, adhäsiv an die darunterliegende Schicht gebunden und/oder adhäsiv in die Schicht eingelagert, kontinuierlich und in geringen Dosen freigesetzt wird, so dass nicht die Besiedelung der Stentoberfläche mit Zellen unterbunden, jedoch eine Überbesiedelung verhindert wird. Diese Kombination beider Effekte verleiht dem erfindungsgemäßen Stent die Fähigkeit, schnell in die Gefäßwand einzuwachsen und vermindert sowohl das Risiko einer Restenose als auch das Risiko einer Thrombose. Die Freisetzung des oder der Wirkstoffe erstreckt sich über einen Zeitraum von 1 bis 24 Monate, bevorzugt über 1-12 Monate nach Implantation, besonders bevorzugt 1-3 Monate nach Implantation.
Die Freisetzung des Wirkstoffes läßt sich über die Regulation der Porengröße mit dem Zusatz des Polyvinylpyrrolidons oder eines ähnlichen hydrophilen Polymeren so adaptieren, das den individuellen Charakteristika des Wirkstoffes, die Elutionsrate als auch seine Pharmakokinetik und bei mehr als einem Wirkstoff auch die Elutionsreihenfolge den benötigten Erfordernissen entsprochen werden kann.

Als Wirkstoffe werden antiproliferative Substanzen, antiphlogistische als auch antithrombotische Stoffe eingesetzt. Als antiproliferative Wirkstoffe werden bevorzugt Cytostatika, Makrolidantibiotika, und/oder Statine eingesetzt. Anwendbare antiproliferative Wirkstoffe sind Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid Estramustin, Melphalan, Betulinsäure, Camptothecin, Lapachol, β-Lapachon, Podophyllotoxin, Betulin, Tropfosfamid, Podophyllsäure-2-ethylhydrazid, Ifosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Mofebutazon, Acemetacin, Diclofenac, Lonazolac Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense Selectin (Cytokinantagonist) CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, Folsäure und Derivate, Vitamine der B-Reihe, Vitamin D-Derivate wie z.B. Calcipotriol und Tacalcitol, Thymosin α-1, Fumarsäure und ihre Derivate wie z.B. Dimethylfumarat, IL-1β-Inhibitor, Colchicin, NO-Donoren wie Pentaerythrityltetranitrat und Syndnoeimine, S-Nitrosoderivate, Tamoxifen, Staurosporin, β-Estradiol, α-Estradiol, Estron, Estriol, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate (6-α-Hydroxy-Paclitaxel, Baccatin, Taxotere u.a.), synthetisch hergestellte als auch aus nativen Quellen gewonnene macrocyclische Oligomere des Kohlensuboxids (MCS) und seine Derivate, Molgramostim (rhuGM-CSF), Peginterferon α-2b, Lanograstim (r-HuG-CSF), Filgrastim, Macrogol, Dacarbazin, Basiliximab, Daclizumab, Ellipticin, D-24851 (Calbiochem), Colcemid, Cytochalasin A-E, Indanocine, Nocadazole, S 100 Protein, Pl-88, Melanocyte Stimulating Hormon (α-MSH), Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1 genannt. Aus der Gruppe der Antibiotika finden des weiteren Cefadroxil, Cefazolin, Cefaclor, Cefotixin Tobramycin, Gentamycin Anwendung. Positiven Einfluß auf die postoperative Phase haben auch Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, Hemoparin® (desulfatiertes und N-reacetyliertes Heparin), Gewebe-Plasminogen-Aktivator, GpIIb/IIIa-Plättchenmembranrezeptor, Faktor Xₐ-Inhibitor, aktiviertes Protein C, Antikörper, Heparin, Hirudin, r-Hirudin, PPACK, Protamin, Prourokinase, Streptokinase, Warfarin, Urokinase, Vasodilatoren wie Dipyramidol, Triazolopyrimidine (Trapidil^{®}), Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Caspaseinhibitoren, Apoptoseinhibitoren, Apoptose-regulatoren wie p65, NF-kB und Bcl-xL-Antisense-Oligonukleotiden und Prostacyclin, Vapiprost, α,β- und γ-Interferon, Histaminantagonisten, Serotoninblocker, Halofuginon, Nifedipin, Tocopherol, Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procäinimid, Retinolsäure, Quinidin, Disopyrimid, Flecainid, Propafenon, Sotolol, Amidoron. Weitere Wirkstoffe sind Steroide (Hydrocortison, Betamethason, Dexamethason), nichtsteroidale Substanzen (NSAIDS) wie Fenoporfen, Ibuprofen, Indomethacin, Naproxen, Phenylbutazon und andere. Antivirale Agentien wie Acyclovir, Ganciclovir und Zidovudin sind ebenfalls einsetzbar. Verschiedene Antimykotika finden Anwendung in diesem Bereich. Beispiele sind Clotrimazol, Flucytosin, Griseofulvin, Ketoconazol, Miconazol, Nystatin, Terbinafin. Antiprozoale Agentien wie Chloroquin, Mefloquin, Quinin sind gleichermassen wirksame Agentien, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3, Tubeimosid, Bruceanole A,B,C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, 1,11-Dimethoxycanthin-6-on, 1-Hydroxy-11-Methoxycanthin-6-on, Scopolectin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-4,16-dien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, lsobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychno-pentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B, weitere natürliche Terpenoide wie Hippocaesculin, 14-Dehydroagrostistachin, C-Type Natriuretic Peptide (CNP), Agroskerin, Agrostistachin, 17-Hydroxyagrostistachin, Ovatodiolide, 4,7-Oxy-cycloanisomelsäure, Yadanzioside N und P, Isodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin.

Die Wirkstoffe werden einzeln oder kombiniert in gleicher oder unterschiedlicher Konzentration eingesetzt. Besonders bevorzugt sind Wirkstoffe, welche neben ihrer antiproliferativen Wirkung auch immunsuppressive Eigenschaften aufweisen. Zu derartigen Wirkstoffen zählen Erythromycin, Midecamycin, Tacrolimus, Sirolimus, Paclitaxel und seine Derivate und Josamycin. als auch Triazolopyrimidine (Trapidil®), D-24851, α- und β-Estradiol, Macrocyclisches Kohlensuboxid (MCS) und dessen Derivate, Pl-88, Natriumsalz der 2-Methylthiazolidin-1,4-dicarbonsäure und Derivate, und Sirolimus.
Zudem bevorzugt ist eine Kombination von mehreren antiproliferativ wirkenden Substanzen oder von antiproliferativen Wirkstoffen mit immunsuppressiven Wirkstoffen.

Insbesondere bevorzugt sind die Wirkstoffe ausgewählt aus der Gruppe umfassend: Paclitaxel und seine Derivate, β-Estradiol, Simvastatin, PI 88 (Sulfatiertes Oligosaccharid; Progen Ind.), Macrocyclische Kohlensuboxide (MCS) und deren Derivate, Trapidil^{®}, N-(Pyridin-4-yl)-[1-4-(4-chlorobenzyl)-indol-3-yl]-glyoxylamid (D-24851), Tacrolimus handelt.

Der Wirkstoff ist bevorzugt in einer pharmazeutisch aktiven Konzentration von 0,001 - 20 mg pro cm² Stentoberfläche, weiter bevorzugt von 0,005 - 15 und insbesondere bevorzugt von 0,01 - 10 mg pro cm² Stentoberfläche enthalten. Weitere Wirkstoffe können in ähnlicher Konzentration in derselben oder in weiteren Schichten enthalten sein. Bevorzugt ist auch eine Ausführungsform, welche zwei verschiedene Wirkstoffe in derselben Schicht oder in unterschiedlichen Schichten enthält. Ferner ist eine Ausführungsform bevorzugt, welche als oberste Schicht eine reine Wirkstoffschicht aufweist.

Die aufgebrachten Polymermengen betragen pro hemokompatiblem Medizinprodukt und insbesondere pro Stent pro Schicht bevorzugt zwischen 0,01 mg/cm² bis 3 mg/cm² Oberfläche, weiter bevorzugt 0,20 mg bis 1 mg und insbesondere bevorzugt 0,2 mg bis 0,5 mg/cm² Oberfläche.

Bevorzugt sind zudem Ausführungsformen, welche in zwei Schichten einen Wirkstoff enthalten. Dies können auch zwei unterschiedliche Wirkstoffe sein. Ist derselbe Wirkstoff in zwei Schichten enthalten, so ist bevorzugt, wenn die beiden Schichten eine unterschiedliche Konzentration an Wirkstoff aufweisen. Des weiteren ist bevorzugt, wenn die untere Schicht eine geringere Wirkstoffkonzentration als die obere Schicht aufweist.

Die erfindungsgemäßen Stents lassen sich durch ein Verfahren zur biokompatiblen Beschichtung von Stents herstellen, dem folgendes Prinzip zugrunde liegt:
a. Bereitstellen eines Stents, und
b. Aufbringen mindestens einer biostabilen Schicht aus Polysulfon mit oder ohne mindestens eines hydrophilen Polymeren, und
c. Auf- und/oder Einbringen mindestens eines antiproliferative, antiinflammatorische, antiphlogistische und/oder antithrombotische Wirkstoffs auf und/oder in die biostabile Schicht, oder
b'. Aufbringen mindestens einer biostabilen Schicht aus Polysulfon mit oder ohne dem mindestens einen hydrophilen Polymeren zusammen mit mindestens einem antiproliferative, antiinflammatorische, antiphlogistische und/oder antithrombotische Wirkstoff.

Nach dem Schritt b' kann vorzugsweise noch der Schritt c' folgen:
c'. Aufbringen von mindestens einem antiproliferative, antiinflammatorische, antiphlogistische und/oder antithrombotische Wirkstoff auf die biostabile Polymerschicht.

Nach den Schritten a, b und c oder den Schritten a, b' oder den Schritten a, b' und c' kann noch ein weiterer Schritt d folgen:
d. Aufbringen mindestens einer zweiten biostabilen Schicht aus Polysulfon.

Diese zweite biostabile Schicht aus Polysulfon kann zum einen aus einem anderen Polysulfon als die erste darunterliegende Schicht bestehen und zum anderen eine andere Menge desselben oder eines anderen hydrophilen Polymeren enthalten. Bevorzugt ist, wenn diese zweite biostabile Schicht aus Polysulfon mindestens einen Wirkstoff enthält. Insbesondere bevorzugt sind Ausführungsformen mit einer biostabilen Schicht aus Polysulfon mit oder ohne hydrophilem Polymeren als äussere Schicht.

Der antiproliferative, antiinflammatorische, antiphlogistische und/oder antithrombotische Wirkstoff wird bevorzugt aus der oben aufgeführten Gruppe ausgewählt.

Ferner sind Ausführungsformen bevorzugt, welche eine hemokompatible Schicht aufweisen. Diese hemokompatible Schicht besteht aus den oben genannten hemokompatiblen Substanzen, insbesondere aus vollständig desulfatiertem und N-reacetyliertem Heparin, desulfatiertem und N-reacetyliertem Heparin, N-carboxymethyliertem, partiell N-acetyliertem Chitosan und/oder aus Mischungen dieser Substanzen und wird unmittelbar oder mittelbar auf die untere biostabile Schicht aufgebracht. Diese hemokompatible Schicht kann sich zwischen zwei anderen Schichten befinden als auch die oberste Schicht bilden. Ausführungsformen mit zwei hemokompatiblen Schichten sind auch möglich, wobei aber nur eine hemokompatible Schicht bevorzugt ist. Die hemokompatible Schicht kann adhäsiv als auch kovalent oder zum Teil adhäsiv und zum Teil kovalent an die darunterliegende Schicht gebunden werden.

Die jeweiligen Schichten werden bevorzugt im Tauch- oder Sprühverfahren aufgebracht. Zudem werden die einzelnen Schichten bevorzugt erst dann auf die darunterliegende Schicht aufgebracht, wenn diese trocken ist.

Bevorzugt ist ein Verfahren, welches aus den beiden Schritten a) und b') besteht.

Das Beschichtungsprinzip bietet eine große Variationsbreite bezüglich der gestellten Anforderungen an den Wirkstoff und auch an die Eigenschaften des eingesetzten Polysulfons, so dass sich verschiedene Beschichtungsvarianten ergeben, die ebenfalls untereinander kombinierbar sind. Die Möglichkeit die Eigenschaften des Polysulfons über die Menge und Molekulargewicht des zugegebenen hydrophilen Polymer wie PVP zu beeinflussen, stellt bezüglich der eingesetzten Wirkstoffe ein weites Feld der Anpassungsfähigkeit der Komponenten zu einem aufeinander abgestimmten System dar.

Weitere Schichten aus Polysulfon ohne Zusatz von PVP und/oder mit gleichem oder sich unterscheidendem PVP-Gehalt mit und ohne Wirkstoffe sind möglich. Desgleichen lässt sich eine direkt an die Stentoberfläche bevorzugt kovalent gebundene Schicht aus vollständig N-deacetyliertem und reacetyliertem Heparin, desulfatiertem und N-reacetyliertem Heparin, N-carboxymethyliertem und/oder partiell N-acetyliertem Chitosan und/oder aus Mischungen dieser Substanzen aufbringen, deren athrombogene Eigenschaften bei Verletzung der darüberliegenden biostabilen Schicht oder Schichten, wie sie zum Beispiel im Vorfeld oder auch während der Implantation durch mechanische Zerstörung der Beschichtung entstehen können für die Maskierung der darunterliegenden Fremdoberfläche sorgen. Diese inerte Schicht lässt sich im Bedarfsfall wahlweise kovalent oder adhäsiv ebenfalls zwischen zwei Schichten und/oder als Toplayer einsetzen.

### Variante A:

a.) Bereitstellen eines unbeschichteten Stents
b.) Aufbringen einer biostabilen Polysulfon-Schicht mit oder ohne hydrophilem Polymer
c.) Aufbringen eines Wirkstoffes oder Wirkstoffkombination in und/oder auf die Polysulfonschicht im Tauch- oder Sprühverfahren
d.) im wesentlichen vollständiges und/oder unvollständiges Überziehen der biostabilen wirkstoffenthaltenden Polysulfon-Schicht mit mindestens einer weiteren biostabilen Polysulfon-Schicht, die der ersten Schicht entspricht oder sich von dieser ersten Schicht in ihrem Gehalt an hydrophilem Polymer und damit in der Porengröße unterscheidet
e.) Aufbringen des selben oder eines weiteren Wirkstoffes oder einer Wirkstoffkombination in und/oder auf die äußere biostabile Schicht, so dass gezielt unterschiedliche Wirkstoffe und/oder Wirkstoffkombinationen mit Hilfe der beiden Schichten voneinander getrennt auf den Stent gebracht werden können, als auch bei unterschiedlicher Porengröße des Polymeren eine unterschiedliche Beladung mit Wirkstoff realisierbar ist als auch eine unterschiedliche Elutionsgeschwindigkeit desselben und/oder eines weiteren Wirkstoffes ermöglicht wird.

Insbesondere bedeutet der Begriff "Aufbringen" in Schritt c) und/oder Schritt e) "Diffusion" des Wirkstoffs in die jeweilige Schicht.

Bevorzugt sind Medizinprodukte mit zwei biostabilen Schichten aus Polysulfon, welche unterschiedliche hydrophile Polymere in unterschiedlichen Konzentrationen enthalten können.

Ebenfalls durchführbar ist das Aufbringen aller vorgesehenen Polymerschichten vor Diffusion des Wirkstoffes in diese Schichten, wenn derselbe Wirkstoff oder Wirkstoffkombination sich in beiden Schichten wiederfinden soll.
Zusätzlich lässt sich eine weitere Schicht eines geeigneten Polysulfons oder auch des reinen hydrophilen Polymeren als Diffusionsbarriere und Topcoat aufbringen.

### Variante B

a.) Bereitstellen eines unbeschichteten Stents
b.) Aufbringen einer biostabilen Polysulfon-Schicht mit oder ohne hydrophilem Polymeren
c.) im wesentlichen vollständiges und/oder unvollständiges Überziehen der biostabilen Polymer-Schicht mit mindestens einem antiproliferativen, antiphlogistischen und/oder antithrombotischen Wirkstoff und/oder Wirkstoffkombination im Sprühverfahren
d.) im wesentlichen vollständiges und/oder unvollständiges Überziehen der Wirkstoffschicht mit mindestens einer weiteren biostabilen Polysulfon-Schicht, die der ersten Schicht entspricht oder sich von dieser ersten Schicht in ihrem Gehalt an hydrophilem Polymer und damit in der Porengröße unterscheidet mit oder ohne Wirkstoff und/oder Wirkstoffkombination
   und/oder
d'.) im wesentlichen vollständiges und/oder unvollständiges Überziehen der Wirkstoffschicht mit einem hydrophilen Polymeren als Topcoat mit oder ohne Wirkstoff und/oder Wirkstoffkombination

Mit diesen Varianten vermag man, das Beschichtungsmaterial an die Wirkstoffe und auch die zeitlich freiwerdende Wirkstoffmenge an die Erfordernisse an dem betroffenen Segment anzupassen.
Bei Mehrschichtsystemen überdeckt die neu aufgebrachte Schicht die darunterliegende Schicht im wesentlichen vollständig. "Im wesentlichen" bedeutet zu 50 bis 100%, bevorzugt zu 70 - 100%, weiter bevorzugt zu 80 - 100%, weiter bevorzugt zu 90 - 100% und insbesondere bevorzugt zu über 96% und insbesondere weiter bevorzugt zu über 98%.

Gegenstand der Erfindung sind auch die nach den vorgenannten Verfahren herstellbaren hemokompatiblen Medizinprodukte und insbesondere hemokompatible Stents.

Die erfindungsgemäßen hemokompatiblen Stents lösen sowohl das Problem der akuten Thrombose als auch das Problem der Neointimahyperplasie nach einer Stentimplantation. Zudem eignen sich die erfindungsgemäßen hemokompatiblen Stents aufgrund ihrer Beschichtung, ob als Einzelschicht oder als Mehrschichtsystem, besonders gut zur kontinuierlichen Freisetzung eines oder mehrerer antiproliferative, antiinflammatorische, antiphlogistischen, antithrombotische und/oder immunsuppressiver Wirkstoffe. Aufgrund dieser Fähigkeit der gezielten kontinuierlichen Wirkstofffreisetzung in erforderlicher Menge verhindern die erfindungsgemäß beschichteten Stents die Gefahr der Restenose fast vollständig.

Die Verhinderung oder Reduzierung der Restenose erfolgt einerseits durch Unterdrückung der zellulären Reaktionen in den ersten Tagen und Wochen nach Implantation mit Hilfe der ausgewählten Wirkstoffe und Wirkstoffkombinationen und andererseits durch die Bereitstellung einer biokompatiblen Oberfläche, so dass mit Abklingen des Wirkstoffeinflusses keine Reaktionen auf die bestehende Fremdoberfläche mehr einsetzen, die langzeitlich ebenfalls zu einem Wiederverschluß des Blutgefäßes führen würden.

### Figurenbeschreibung

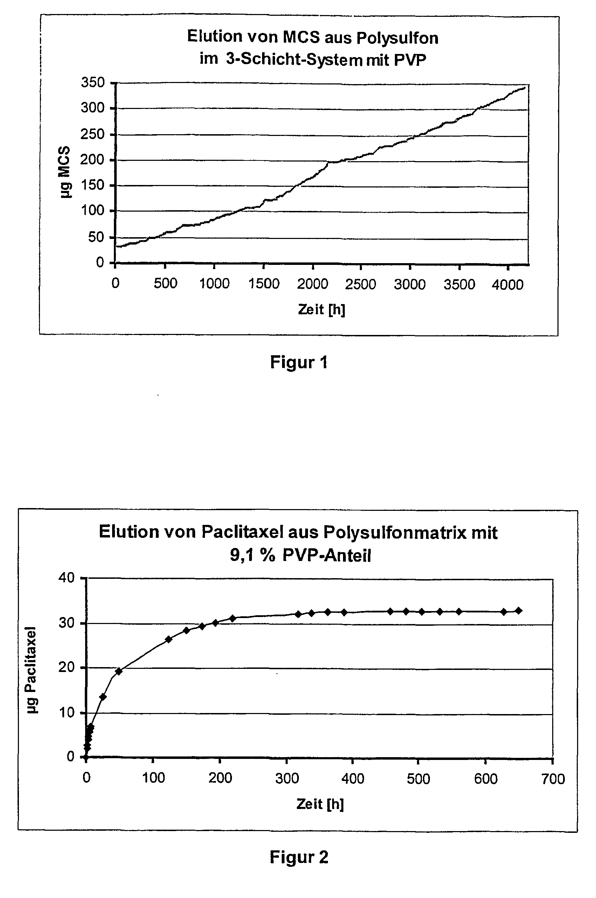

- Figur 1:: Elutionsdiagramm von Macrocyclischem Kohlensuboxid (MCS) in einem Dreischichtsystem mit Polysulfon als Basisbeschichtung, dem Wirkstoff als Mittelschicht und einer die mittlere Wirkstoffschicht vollständig bedeckende Beschichtung von Polysulfon mit einem Anteil von 0,04% Polyvinylpyrrolidon.
- Figur 2:: Elutionsdiagramm von Paclitaxel aus einer Polysulfonmatrix mit einem Anteil von 9,1% Polyvinylpyrrolidon

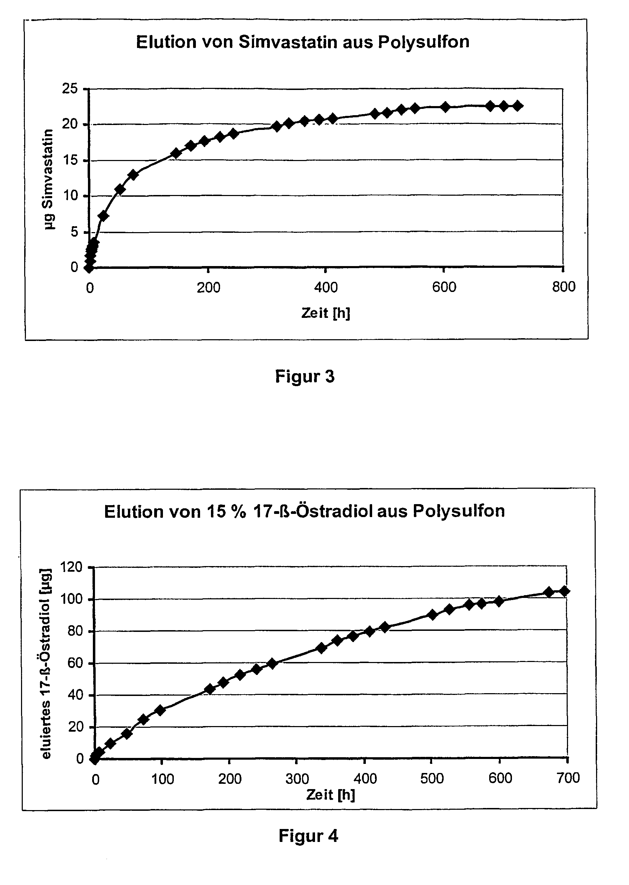

- Figur 3:: Elutionsdiagramm von Simvastatin aus reiner Polysulfonmatrix ohne Anteil von hydrophilem Polymer
- Figur 4:: Elutionsdiagramm von β-Estradiol mit anteilsmäßig 15 Gew.-% an der reinen Polysulfonmatrix ohne Anteil von hydrophilem Polymer

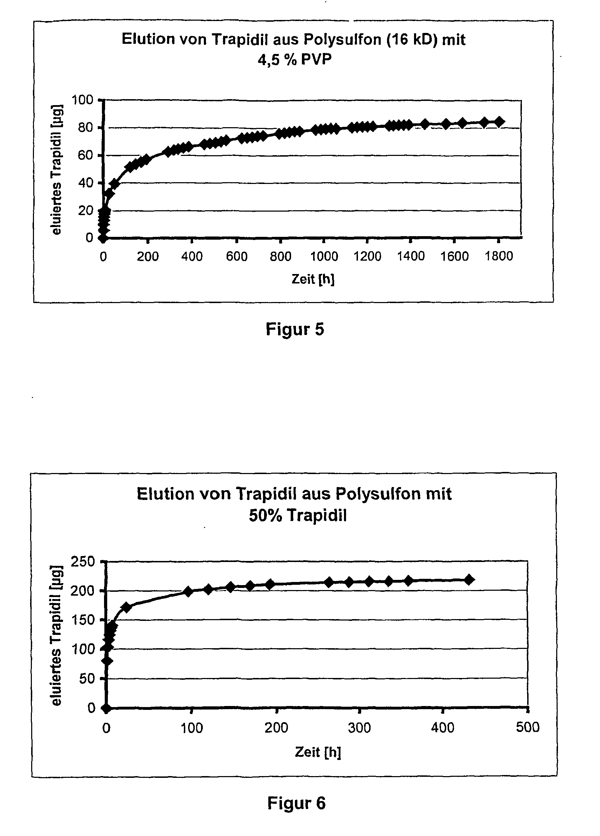

- Figur 5:: Elutionsdiagramm von Trapidil^{®} aus einer Polysulfonmatrix mit einem Anteil von 4,5% Polyvinylpyrrolidon
- Figur 6:: Elutionsdiagramm von Trapidil^{®} mit einem 50% Anteil an der reinen Polysulfonmatrix

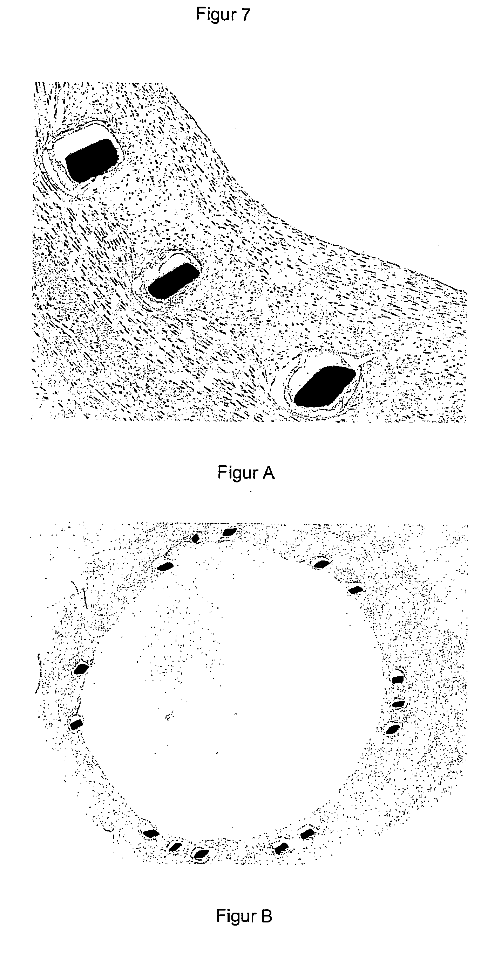

- Figur 7:: Photomicrographie der Gefässegmente nach 4 Wochen Implantation im Schwein.
Bild A ist ein vergrösserter Ausschnitt eines Matrixstentes
Billd B zeigt einen Querschnitt durch das Gefäßsegment mit dem in höherer Konzentration mit MCS beladenen Polysulfonbeschichteten Stent

### Beispiele

### Beispiel 1

### Beschichtung von Stents mit Polyethersulfon

### Sprühlösung:

a. PS- Lösung:
176 mg PS (Polyethersulfon, Udel®, erhältlich von Solvay) werden eingewogen und mit Chloroform auf 20 g aufgefüllt.
→ 0,88 % PS

| | Sprühlösung | vor | nach | Masse |
|---|---|---|---|---|
| Stent | | Beschichtung | Beschichtung | Beschichtung |
| 1 | 2,0 ml | 0,01754 g | 0,01826 g | 0,72 mg |
| 2 | 2,0 ml | 0,01814 g | 0,01889 g | 0,75 mg |
| 3 | 2,0 ml | 0,01751 g | 0,01832 g | 0,81 mg |
| 4 | 2,0 ml | 0,01742 g | 0,01816 g | 0,74 mg |
| 5 | 2,0 ml | 0,01734 g | 0,01814 g | 0,80 mg |
| 6 | 2,0 ml | 0,01736 g | 0,01815 g | 0,80 mg |

### Beispiel 2

### Beschichtung von Stents mit Polyethersulfon (Basiscoat) und Polyethersulfon mit 0,04% PVP bzw. 0,08% PVP als Topcoat

### Sprühlösungen:

a. Polysulfon - Lösung:
   17,6 mg PS werden eingewogen und mit Chloroform auf 2 g aufgefüllt. → 0,88 % PS
b. Polysulfon / PVP - Lösung:
   25,2 mg PS und 1,2 mg PVP werden eingewogen und mit Chloroform auf 3 g aufgefüllt.
   → 0,84 % PS, 0,04 % PVP
b'. Polysulfon / PVP - Lösung:
   24 mg PS und 2,4 mg PVP werden eingewogen und mit Chloroform auf 3 g aufgefüllt.
   → 0,80 % PS, 0,08 % PVP

### Sprühbeschichtung:

Die gewogenen Stents werden mit den Sprühlösungen in der angegebenen Reihenfolge mit a.) 0,5 ml und b.) 0,85 ml sprühbeschichtet. Dabei wird nach jedem Sprühvorgang mindestens 6 Stunden gewartet, ehe die nächste Schicht aufgebracht wird. Nach dem Trocknen bei Raumtemperatur über Nacht im Reinraum wird erneut gewogen.

| Stent | vor Beschichtung | nach Beschichtung | Masse Beschichtung |
|---|---|---|---|
| 1b | 0,02058g | 0,02132g | 0,75 mg |
| 1b' | 0,01968g | 0,02022g | 0,54 mg |
| 2b' | 0,01968g | 0,02034g | 0,66 mg |

### Beispiel 3

### Herstellung von Stents mit MCS und Polyethersulfon im 3-Schicht-System nach Variante B

### Sprühlösungen:

a) Polyethersulfon - Lösung: (erste Schicht : Basecoat, Basiscoat) :
   70,4 mg PS werden eingewogen und mit Chloroform auf 8 g aufgefüllt. → 0,88 % PS
b) MCS - Lösung (2. Schicht: Middtecoat):
   39,6 mg MCS werden eingewogen und mit 20 % Ethanol in Wasser auf 18 g aufgefüllt.
   → 0,22 % MCS
c) Polyethersulfon / PVP- Lösung (3.Schicht : Topcoat):
   100,8 mg PS und 4,8 mg Polyvinylpyrrolidon werden eingewogen und mit Chloroform auf 12 g aufgefüllt.
   → 0,84 % PS, 0,04 % PVP

### Sprühbeschichtung:

Nicht expandierte Edelstahl-Stents werden nach ihrer Reinigung gewogen und sprühbeschichtet. Die Stents werden mit der entsprechenden Menge der jeweiligen Sprühlösung mit a) 0,5 ml; b.) 1,5 ml und c.) 0,85 ml in der angegebenen Reihenfolge besprüht. Dabei wird nach jeder Schicht mindestens 6 Stunden gewartet, ehe die nächste Schicht gesprüht wird. Nach dem Trocknen bei Raumtemperatur über Nacht wird erneut gewogen. Der Mittelwert des Wirkstoffgehaltes auf den Stents beträgt 153 ± 9 µg.

| Stent | vor Beschichtung | nach Beschichtung | Masse Beschichtung | Masse MCS |
|---|---|---|---|---|
| 1 | 0,01829 g | 0,01894 g | 0,65 mg | 141 µg |
| 2 | 0,01753 g | 0,01826 g | 0,73 mg | 159 µg |
| 3 | 0,01772 g | 0,01836 g | 0,64 mg | 139 µg |
| 4 | 0,01719 g | 0,01790 g | 0,71 mg | 154 µg |
| 5 | 0,01833 g | 0,01903 g | 0,70 mg | 152 µg |
| 6 | 0,01774 g | 0,01836 g | 0,62 mg | 135 µg |
| 7 | 0,01729 g | 0,01802 g | 0,73 mg | 159 µg |

### Beispiel 4

### Bestimmung der Elutionskinetik: von MCS aus Polyethersulfon mit 4,5 % PVP

Je ein Stent wird in ein Schnappdeckelgläschen gegeben, mit 2 ml PBS-Puffer versetzt, mit Parafilm verschlossen und für gegebene Zeiten im Trockenschrank bei 37 °C inkubiert. Nach Ablauf der gewählten Zeit wird der Überstand abpipettiert und dessen UV-Absorption bei 207 nm gemessen. Der jeweilige Stent wird erneut mit 2 ml PBS versetzt und wieder bei 37°C inkubiert. Dieser Vorgang wird mehrfach wiederholt.

### Beispiel 5

### Beschichtung von Stents mit Simvastatinbeladener Polysulfonmatrix

### Sprühlösungen:

a. PS / Simvastatin-Lösung:
26,4 mg PS und 8,8 mg Simvastatin werden eingewogen und mit Chloroform auf 4 g aufgefüllt.
→ 0,66 % PS, 0,22 % Simvastatin
b. PS / Simvastatin/PVP-Lösung:
24,8 mg PS, 8,8 mg Simvastatin und 1,6 mg PVP werden eingewogen und mit Chloroform auf 4 g aufgefüllt.
→ 0,62 % PS, 0,22 % Simvastatin, 0,04 % PVP

| Stent | Sprühlösung | vor Beschichtung | nach Beschichtung | Masse Beschichtung | Masse Simvastatin |
|---|---|---|---|---|---|
| 1 | 2,0 ml a) | 0,02164 g | 0,02171 g | 1,08 mg | 270 µg |
| 2 | 2,0 ml b) | 0,02129 g | 0,02253 g | 1,24 mg | 310 µg |

### Beispiel 6

### Beschichtung von Stents mit Simvastatinbeladener Polysulfonmatrix mit hohem PVP-Anteil

### Sprühlösung:

a. PS / Simvastatin/PVP-Lösung:
23,2 mg PS, 8,8 mg Simvastatin und 3,2 mg PVP werden eingewogen und mit Chloroform auf 4 g aufgefüllt.
→ 0,58 % PS, 0,22 % Simvastatin, 0,08 % PVP

| Stent | Sprühlösung | vor Beschichtung | nach Beschichtung | Masse Beschichtung | Masse Simvastatin |
|---|---|---|---|---|---|
| 1 | 2,0 ml a) | 0,02164 g | 0,02171 g | 1,08 mg | 270 µg |
| 2 | 2,0 ml a) | 0,02129 g | 0,02253 g | 1,24 mg | 310 µg |

### Beispiel 7

### Beschichtung von Stents mit Paclitaxelbeladener Polysulfonmatrix- Matrix

### Sprühlösungen:

a. PS / Paclitaxel-Lösung:
13,2 mg PS und 4,4 mg Paclitaxel werden eingewogen und mit Chloroform auf 2 g aufgefüllt.
→ 0,66 % PS, 0,22 % Paclitaxel
b. PS / PVP / Paclitaxel-Lösung:
11,6 mg PS, 1,6 mg PVP und 4,4 mg Paclitaxel werden eingewogen und mit Chloroform auf 2 g aufgefüllt.
→ 0,58 % PS, 0,08 % PVP, 0,22 % Paclitaxel

| Stent | Sprühlösung | Vor Beschichtung | nach Beschichtung | Masse Beschichtung | Masse Paclitaxel |
|---|---|---|---|---|---|
| 1 | 1,0 ml a) | 0,01725 g | 0,01770 g | 0,45 mg | 113 µg |
| 2 | 1,0 ml b) | 0,01735 g | 0,01790 g | 0,55 mg | 138 µg |

### Beispiel 8

### Beschichtung von Stents mit 17-β-Estradiol in Polysulfonmatrix

### Sprühlösungen:

a. PS / 25 % 17-β-Estradiol - Lösung:
46,2 mg PS und 15,4 mg 17-β-Estradiol werden eingewogen und mit Chloroform auf 7 g aufgefüllt.
→ 0,66 % PS, 0,22 % 17-β-Estradiol
b. PS / 20 % 17-β-Estradiol- Lösung:
28,2 mg PS und 7 mg 17-β-Estradiol werden eingewogen und mit Chloroform auf 4 g aufgefüllt.
→ 0,704 % PS, 0,176 % 17-β-Estradiol
c. PS / 15 % 17-β-Estradiol - Lösung:
29,9 mg PS und 5,3 mg 17-β-Estradiol werden eingewogen und mit Chloroform auf 4 g aufgefüllt.
→ 0,748 % PS, 0,132 % 17-β-Estradiol

| | Sprühlösung | vor | nach | Masse | Masse |
|---|---|---|---|---|---|
| Stent | | Beschichtung | Beschichtung | Beschichtung | 17-β-Estradiol |
| 1 | 2,2 ml a) | 0,02052 g | 0,02166 g | 1,14 mg | 285 µg |
| 2 | 2,2 ml a) | 0,02065 g | 0,02189 g | 1,24 mg | 310 µg |
| 3 | 2,2 ml b) | 0,02080 g | 0,02206 g | 1,27 mg | 254 µg |
| 4 | 2,2 ml c) | 0,02064 g | 0,02213 g | 1,49 mg | 224 µg |

### Beispiel 9

### Beschichtung von Stents mit einer Triazolopyrimidine (Trapidil^{®}) enthaltenden Polysulfonmatrix

### Sprühlösung:

PS / Trapidil^{®} - Lösung:
19,8 mg PS und 6,6 mg Trapidil^{®} werden eingewogen und mit Chloroform auf 3 g aufgefüllt.
→ 0,66 % PS, 0,22 % Trapidil^{®}

| Stent | Sprühlösung | vor Beschichtung | nach Beschichtung | Masse Beschichtung | Masse Trapidil^{®} |
|---|---|---|---|---|---|
| 1 | 1,7 ml | 0,01742 g | 0,01855 g | 1,13 mg | 283 µg |

### Beispiel 10

### in vivo-Untersuchungen von Stents mit Polyethersulfon als Matrix mit und ohne Macrocyclischem Suboxid

In 13 Hausschweine unterschiedlichen Geschlechts mit 20-25kg Gewicht wurden in die Koronararterien mit Polyethersulfon beschichtete Stents implantiert. Es wurde zwischen drei Gruppen von Stents unterschieden. Eine Gruppe enthielt eine hohe Dosierung Paclitaxel, die zweite enthielt eine niedrige Dosierung Paclitaxel und die letzte Gruppe war der reine Matrixstent ohne Wirkstoffzusatz. Nach vier Wochen wurden die Stents entfernt und auf Entzündungsreaktionen (peri-strut) und Neointimabildung untersucht.

| Histomorphometrische Evaluierung nach 4 Wochen Implantationszeit | | | | |
|---|---|---|---|---|
| Beschichtung | Anzahl Stents | Intima-Dicke | Stenose | Verletzungs- |
| | | [mm] | [%] | grad |
| Matrix/hohe Wirkstoffkonzentration | 6 | 0,14±0,06 | 19±9 | 0,32±0,19 |
| Matrix/niedrige Wirkstoffkonzentration | 6 | 0,23±0,07 | 32±10 | 0,46±0,29 |
| Matrix ohne Wirkstoff | 4 | 0,17±0,06 | 23±8 | 0,15±0,12 |

Alle untersuchten Stents unabhängig von der Beschichtung zeigten nur minimale Entzündungen um die Stentstreben und an der Adventitia. Die höhere durchschnittliche Intima-Dicke der Stents mit der niedrigen Wirkstoffbeladung liessen sich auf die während der Implantation stärkeren Überdehnung des Gefässes zurückführen. Der reine Matrixstent zeigt keine dem Polymer zuzuordnende Auffälligkeiten in den Gefässreaktionen, was seine Hämokompatiblität und Eignung als Wirkstoffträger unterstreicht.

### Beispiel 11

### in vivo-Untersuchungen von Stents mit Polyethersulfon als Matrix mit und ohne Paclitaxel

Analog dem vorhergehenden Beispiel 10 wurden mit Polyethersulfon beschichtete Stents mit Paclitaxel-beladenen Polyethersulfonbeschichteten Stents verglichen:

| Histomorphometrische Evaluierung nach 4 Wochen Implantationszeit | | | | |
|---|---|---|---|---|
| Beschichtung | Anzahl Stents | Intima-Dicke | Stenose | Verletzungs- |
| | | [mm] | [%] | grad |
| Matrix/Wirkstoff | 6 | 0,21±0,10 | 26±12 | 0,23±0,20 |
| Matrix | 4 | 0,14±0,06 | 18±8 | 0,10±0,07 |

Auch die Ergebnisse dieser Studie zeigen den Erfolg der Polysulfonbeschichtung.

### Beispiel 12

### Herstellung des Polysulfons gemäß Formel (IIA).

Das Polysulfon (IIA) wurde nach der Vorschrift von E. Avram et al. J. Macromol Sci. Pure Appl. Chem., 1997, A34, 1701 hergestellt.
3 Molequivalente Benzylalkohol werden in Toluol gelöst und mit Natrium deprotoniert. 1 Molequivalent des Polysulfons (IIA) wird zugegeben und danach wird die Reaktionsmischung auf Siedetemperatur erwärmt.
Das Reaktionsprodukt wird in einer Ausbeute von 22% erhalten.

### Beispiel 13

### Herstellung des Polysulfons gemäß Formel (IIC).

Das Polysulfon (IIC) wurde nach der Vorschrift von M. D. Guiver et al., Brit. Polym. L. 1990, 23, 29 hergestellt.
1 g des erhaltenen Polysulfons (IIC) wurde unter Verwendung von Orthoessigsäureethylester verestert, wobei Toluol als Lösungsmittel eingesetzt wurde und die flüchtigen Reaktionsprodukte mittels Destillation dem Reaktionsgleichgewicht entzogen wurden. 40% der Carboxylatgruppen wurden in Ethylestergruppen überführt.
Gemäß Beispiel 7 wurde dieses Polymer zusammen mit Paclitaxel auf einen Stent aufgebracht.
Der Stent zeigt gute Hämokompatibilität und eine amorphe Polysulfonbeschichtung, welche zur kontrollierten Freisetzung des Paclitaxel geeignet war.

### Beispiel 14

1g des gemäß Beispiel 12 hergestellten Polysulfons wird mit 200 mg des Polysulfons gemäß Formel (IIC) gemischt und gemäß Beispiel 7 zusammen mit dem Wirkstoff Paclitaxel auf einen Stent aufgebracht.
Der beschichtete Stent weist eine gute Hämokompatibilität und eine amorphe Polysulfonbeschichtung auf, welche zur kontrollierten Freisetzung des Paclitaxel geeignet war.

### Beispiel 15

### Einführung von Chorsulfongruppen an Polysulfon

2,4 g Polysulfon wird in 700ml Chloroform gelöst und auf -20°C gekühlt. Anschließend werden 23,3 ml Chlorsulfonsäure langsam zugetropft. Da die Reaktion stark exotherm ist, wird das Reaktionsgefäß im Eisbad gekühlt. Nach Zugabe der Chlorsulfonsäure läßt man die Lösung unter Rühren auf Raumtemperatur erwärmen. Nach 30 Minuten wird das Polymer in Ethanol gefällt und anschließend mit VE-Wasser gespült. Zur vollständigen Entfernung der Chlorsulfonsäure wird nochmals 10 Minuten in VE-Wasser extrahiert.

### Beispiel 16

### S-Alkoxy-de-Chlorierung

10g Ethanol werden in 100ml Wasser gelöst und mit 2-3 Tropfen Methylrot in Aceton versetzt. Diese Lösung wird auf 5 g feinkörnigen chlorsulfonierten Polysulfon gegeben.
Die Lösung wird mit 5N KOH tropfenweise versetzt bis der Farbumschlag von Gelb nach Rot erfolgt. Danach wird das Gefäß verschlossen und gut geschüttelt. Es wird solange Kalilauge zugegeben und geschüttelt bis der Farbumschlag ausbleibt. Der gebildete Polysulfonester wird abgesaugt und mit Wasser gewaschen und zur Reinigug umkristallisiert.

### Beispiel 17

### S-Alkoxy-de-Chlorierung

10g trockener Ethanol werden mit 60ml Pyridin versetzt. Diese Lösung wird unter Eiskühlung zu 40g fein gepulvertem chlorsulfonierten Polysulfon gegeben. Anschließend wird unter Feuchtigkeitsausschluß über Nacht bei Raumtemperatur gerührt. Anschließend wird die Suspension in Eiswasser gegossen und mit konz. Salzsäure vorsichtig angesäuert. Die Wäsche erfolgt mit wäßriger Hydrogencarbonatlösung. Nach dem Abfiltrieren
kann das veresterte Polysulfon umkristallisiert werden.

### Beispiel 18

Beschichtung mit einer Mischung aus Polysulfon und Polysulfon gemäß Formel (IIC) 24 mg PS und 2,4 mg Polysulfon gemäß Formel (IIC) werden eingewogen und mit Chloroform auf 3 g aufgefüllt.
à 0,80 % PS, 0,08 % PVP
Ein Stent wird gemäß Beispiel 7 mit dieser Mischung im Sprühverfahren beschichtet.

## Patentansprüche

1. **Hemokompatibles** Medizinprodukt, **dadurch gekennzeichnet, daß** dessen Oberfläche zumindest teilweise mit mindestens einer biostabilen Schicht aus Polysulfon beschichtet ist und dass sich in, unter und/oder auf der mindestens einen biostabilen Schicht aus Polysulfon mindestens ein antiproliferativer, antiinflammatorischer, antiphlogistischer und/oder antithrombotischer Wirkstoff befindet.

2. **Hemokompatibles** Medizinprodukt nach Anspruch 1 **dadurch gekennzeichnet, daß** das Polysulfon aus der Gruppe ausgewählt wird umfassend: Polyethersulfon, substituiertes Polyethersulfon, Polyphenylsulfon, substituiertes Polyphenylsulfon, Polysulfonblockcopolymere, perfluorierte Polysulfonblockcopolymere, semifluorierte Polysulfonblockcopolymere, substituierte Polysulfonblockcopolymere und/oder Mischungen der vorgenannten Polymere.

3. **Hemokompatibles** Medizinprodukt nach Anspruch 1 oder 2, **dadurch gekennzeichnet, dass** die mindestens eine biostabile Schicht aus Polysulfon mindestens ein hydrophiles Polymer enthält.

4. **Hemokompatibles** Medizinprodukt nach Anspruch 3, **dadurch gekennzeichnet, dass** das Polysulfon mit dem mindestens einen hydrophilen Polymer in einem Mischungsverhältnis von 50 Gew.-% : 50 Gew.-% bis 99,999 Gew.-% : 0,001 Gew.-% vorliegt.

5. **Hemokompatibles** Medizinprodukt nach Anspruch 3 oder 4, **dadurch gekennzeichnet, dass** das hydrophile Polymer aus der Gruppe ausgewählt wird, umfassend: Polyvinylpyrrolidon, Glycerin, Polyethylenglykol, Polypropylenglycol, Polyvinylalkohol, Polyhydroxyethylmethacrylate, Polyacrylamid, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Fibrin, Polycyanoacrylate, Polycaprolactondimethylacrylate, Poly-b-Maleinsäure Polycaprolactonbutyl-acrylate, Multiblockpolymere aus Oligocaprolactondiole und Oligodioxanondiole, Polyetherestermultiblockpolymere aus PEG und Poly(butylenterephtalat), Polypivotolactone, Polyglycolsäuretrimethylcarbonate, Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein; modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Chitosan und seine Derivate, Chondroitinsulfat, Dextrane, b-Cyclodextrine, und Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen.

6. **Hemokompatibles** Medizinprodukt nach Anspruch 5, **dadurch gekennzeichnet, dass** das hydrophile Polymer aus der Gruppe ausgewählt wird, umfassend: Polyvinylpyrrolidon, Polyethylenglykol, Polypropylenglycol und/oder Glycerin.

7. **Hemokompatibles** Medizinprodukt nach einem der vorherigen Ansprüche, **dadurch gekennzeichnet, dass** die Beschichtung der Oberfläche des Medizinprodukts aus einer, zwei, drei oder mehr Schichten besteht.

8. **Hemokompatibles** Medizinprodukt nach einem der vorherigen Ansprüche, **dadurch gekennzeichnet, dass** sich unter und/oder auf der mindestens einen biostabilen Schicht aus Polysulfon mit oder ohne dem mindestens einen hydrophilen Polymer mindestens eine Schicht aus vollständig desulfatiertem und N-reacetyliertem Heparin, desulfatiertem und N-reacetyliertem Heparin, N-carboxymethyliertem und/oder partiell N-acetyliertem Chitosan und/oder aus Mischungen dieser Substanzen befindet.

9. **Hemokompatibles** Medizinprodukt nach einem der vorherigen Ansprüche, **dadurch gekennzeichnet, dass** der mindestens eine antiproliferative, antiinflammatorische, antiphlogistische und/oder antithrombotische Wirkstoffe aus der Gruppe ausgewählt wird umfassend: Sirolimus (Rapamycin), Everolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, C-Type Natriuretic Peptide (CNP), 4-Hydroxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Cryptophycine, Anginex, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, Lapachol, β-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Anginex, Na-Uretic-Peptide, Dacarbazin, Basiliximab, Daclizumab, Selectin (Cytokinantagonist), Chryptophycine, CETP-Inhibitor, Cadherine, Cytokininhibitoren, COX-2-Inhibitor, AE-941 (Neovastat®), NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, Ac-YVAD-CMK, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere u.a., synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertes und N-reacetyliertes Heparin (Hemoparin®), Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren wie Dipyramidol, Triazolopyrimidine (Trapidil®), Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron., natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A,B und C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B umfassen.

10. **Hemokompatibles** Medizinprodukt nach Anspruch 8, **dadurch gekennzeichnet, dass** der mindestens eine antiproliferative, antiinflammatorische, antiphlogistische und/oder antithrombotische Wirkstoffe aus der Gruppe ausgewählt wird umfassend: Paclitaxel und seine Derivate, β-Estradiol, Simvastatin, PI 88 (Progen Ind.), Macrocyclische Kohlensuboxide (MCS) und deren Derivate, Trapidil^{®}, N-(Pyridin-4-yl)-[1-4-(4-chlorobenzyl)-indol-3-yl]-glyoxylamid (D-24851), aktiviertes Protein C (aPC), Ac-YVAD-CMK, Anginex (β-Pep25), Neovastat^{®}, Cryptophycin 52, Tacrolimus handelt.

11. **Hemokompatibles** Medizinprodukt nach einem der vorhergehenden Ansprüche, **dadurch gekennzeichnet, dass** bei Mehrschichtsystemen die mindestens zwei Schichten mit oder/und ohne Zumischung mindestens eines hydrophilen Polymeren mindestens einen Wirkstoff in gleicher oder unterschiedlicher Wirkstoffkonzentration kovalent oder / und adhäsiv gebunden enthalten.

12. **Hemokompatibles** Medizinprodukt nach Anspruch 11,
**dadurch gekennzeichnet, dass** bei Mehrschichtsystemen die letzte Schicht eine reine Wirkstoffschicht kovalent oder / und adhäsiv gebunden ist.

13. **Hemokompatibles** Medizinprodukt nach einen der vorhergehenden Ansprüchen, **dadurch gekennzeichnet, dass** die mindestens eine zumindest einen Wirkstoff enthaltende oder/und mit mindestens einem Wirkstoff überzogene biostabile Polysulfonschicht mindestens teilweise mit einer keinen oder mindestens einen Wirkstoff in gleicher oder unterschiedlicher Konzentration kovalent und/oder adhäsiv enthaltenden biodegradierbaren Polymerschicht überzogen ist.

14. Verfahren zur biokompatiblen und **hemokompatiblen** Beschichtung von Medizinprodukten **gekennzeichnet durch** die Schritte:
a. Bereitstellen eines Stents, und
b. Aufbringen mindestens einer biostabilen Schicht aus Polysulfon mit oder ohne mindestens einem hydrophilen Polymeren, und
c. Auf- und/oder Einbringen mindestens eines antiproliferative, antiinflammatorische, antiphlogistische und/oder antithrombotische Wirkstoffs auf und/oder in die biostabile Schicht, oder
b'. Aufbringen mindestens einer biostabilen Schicht aus Polysulfon mit oder ohne dem mindestens einen hydrophilen Polymeren zusammen mit mindestens einem antiproliferative, antiinflammatorische, antiphlogistische und/oder antithrombotische Wirkstoff und
c'. optional Aufbringen von mindestens einem antiproliferativen, antiinflammatorischen antiphlogistischen und/oder antithrombotischen Wirkstoff auf die biostabile Polymerschicht.

15. Verfahren nach Anspruch 14 umfassend den weiteren Schritt
d'. Aufbringen mindestens einer weiteren Schicht aus mindestens einem biodegradierbarem Polymeren ohne oder mit Einbringen oder/und Aufbringen von mindestens einem Wirkstoff in gleicher oder unterschiedlicher Konzentration.

16. **Hemokompatibles** Medizinprodukt erhältlich nach einem Verfahren gemäß einem der Ansprüche 14 oder 15.

17. **Hemokompatibles** Medizinprodukt nach einem der Ansprüche 1 - 10 oder 16, **dadurch gekennzeichnet, dass** der mindestens eine antiproliferative, antiinflammatorische, antiphlogistische und/oder antithrombotische Wirkstoff kontrolliert durch die Oberflächenbeschichtung freigesetzt wird.

18. **Hemokompatibles** Medizinprodukt nach einem der Ansprüche 1 - 10, 16 oder 17, **dadurch gekennzeichnet, dass** der jeweilige antiproliferative, antiinflammatorische, antiphlogistische und/oder antithrombotische Wirkstoff in einer pharmazeutisch aktiven Konzentration von 0,001 - 10 mg pro cm² Oberfläche des Medizinprodukts und pro wirkstofftragende Schicht enthalten ist.

19. **Hemokompatibles** Medizinprodukt nach einem der Ansprüche 1 - 13 oder 16 - 18, **dadurch gekennzeichnet, dass** es sich bei dem Medizinprodukt um einen Stent handelt.

## Claims

1. Hemocompatible medical product, **characterized in that** its surface is at least partially coated with at least one biostable layer composed of polysulfone and **in that** at least one antiproliferative, anti-inflammatory, antiphlogistic and/or antithrombotic agent is present in, under and/or on the at least one biostable layer composed of polysulfone.

2. Hemocompatible medical product according to claim 1, **characterized in that** the polysulfone is selected from the group comprising: polyethersulfone, substituted polyethersulfone, polyphenylsulfone, substituted polyphenylsulfone, polysulfone block copolymers, perfluorated polysulfone block copolymers, semifluorated polysulfone block copolymers, substituted polysulfone block copolymers and/or mixtures of the aforementioned polymers.

3. Hemocompatible medical product according to claim 1 or 2, **characterized in that** the at least one biostable layer composed of polysulfone contains at least one hydrophilic polymer.

4. Hemocompatible medical product according to claim 3, **characterized in that** the polysulfone and the hydrophilic polymer are present in a mix ratio of 50% per weight: 50% per weight up to 99.999% per weight: 0.001 % per weight.

5. Hemocompatible medical product according to claim 3 or 4, **characterized in that** the hydrophilic polymer is selected from the group comprising: polyvinylpyrrolidone, glycerol, polyethylene glycol, polypropylene glycol, polyvinylalcohol, polyhdroxyethyl methacrylate, polyacrylamide, polyvalerolactone, poly-ε-decalactone, polylactonic acid, polyglycolic acid, polylactides, polyglycolides, copolymers from the polylactides and polyglycolides, poly-ε-caprolactone, polyhydroxybutyric acid, polyhydroxybutyrate, polyhydroxyvalerate, polyhydroxybutyrate-co-valerate, poly(1,4-dioxane-2,3-dione), poly(1,4-dioxane-2,3-dione), poly-paradioxanones, polyanhydrides such as polymaleic acid anhydrides, fibrin, polycyanoacrylate, polycaprolactone dimethylacrylate, poly-β-maleic acid, polycaporlactone butyl acrylate, multiblock polymers from oligocaprolactonediols and oligodioxanonediols, polyetherester multiblock polymers from PEG and poly(butyleneterephtalates), polypivotolactone, polyglycolic acid trimethyl carbonate, polycaprolactone glycolides, poly(γ-ethyl glutamate), poly(DTH-iminocarbonate), poly(DTE-co-DT-carbonate), poly(bisphenol A-iminocarbonate), polyorthoesters, polyglycolic acid trimethyl carbonates, polytrimethyl carbonates, polyiminocarbonates, poly(N-vinyl)-pyrrolidone, polyvinylalcohols, polyesteramides, glycolated polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes having amino acid residues in the backbone, polyetheresters, polyethylene oxide, polyalkene oxalate, polyorthoesters as well as copolymers thereof, lipids, carrageenans, fibrinogen, starch, collagen, protein based polymers, polyamino acids, synthetic polyamino acids, zein, modified zein, polyhydroxyalkanoates, pectic acid, actinic acid, modified and non modified fibrin and casein, carboxymethyl sulfate, albumin, hyaluronic acid, chitosan and derivatives thereof, chondroitin sulfate, dextrans, β-cyclodextrins, and copolymers with PEG and polypropylene glycol, gum arabic, guar, gelatine, collagen-N-hydroxysuccinimide, phospholipids, modifications and copolymers and/or mixtures of the aforementioned substances.

6. Hemocompatible medical product according to claim 5, **characterized in that** the hydrophilic polymer is selected from the group comprising: polyvinylpyrrolidone, polyethylene glycol, polypropylene glycol and/or glycol.

7. Hemocompatible medical product according to any one of the preceding claims, **characterized in that** the coating of the surface of the medical product consists of one, two, three or more layers.

8. Hemocompatible medical product according to any one of the preceding claims, **characterized in that** at least one layer composed of completely desulfated and N-reacetylated heparin, desulfated and N-reacetylated heparin, N-carboxymethylated and/or partially N-acetylated chitosan and/or of mixtures of said substances is present under and/or on the at least one biostable layer composed of polysulfone including or not including the at least one hydrophilic polymer.

9. Hemocompatible medical product according to any one of the preceding claims, **characterized in that** the at least one antiproliferative, anti-inflammatory, antiphlogistic and/or antithrombotic agent is selected from the group comprising: sirolimus (rapamycin), everolimus, somatostatin, tacrolimus, roxithromycin, daunamycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, C-type natriuretic peptide (CNP), 4-hydroxycyclophosphamide, estramustine, melphalan, ifosfamide, trofosfamide, chlorambucil, bendamustine, dacarbazine, busulfan, procarbazine, treosulfan, temozolomide, thiotepa, daunorubicin, doxorubicin, aclarubicin, epirubicin, idarubicin, bleomycin, mitomycin, dactinomycin, methotrexate, fludarabine, fludarabine-5'-dihydrogen phosphate, cladribine, mercaptopurine, thioguanine, cytarabine, fluorouracil, gemcitabine, capecitabine, docetaxel, carboplatin, cisplatin, cryptophycins, anginex, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharanthin, inhibitor 2ω of smc proliferation, epothilone A and B, mitoxantrone, azathioprine, mycophenolate mofetil, c-myc antisense, b-myc antisense, betulinic acid, camptothecin, lapachol, β-lapachone, podophyllotoxin, betulin, podophyllic acid 2-ethylhydrazide, molgramostim (rhuGM-CSF), peginterferon α-2b, lenograstim (r-HuG-CSF), filgrastim, macrogol, basiliximab, daclizumab, selectin (cytokine antagonist), CETP inhibitor, cadherins, cytokinin inhibitors, COX-2 inhibitor, AE-941 (Neovastat^{®}), NFkB, angiopeptin, ciprofloxacin, fluroblastin, monoclonal antibodies inhibiting muscle cell proliferation, bFGF antagonists, probucol, prostaglandins, Ac-YVAD-CMK, 1,11-dimethoxycanthin-6-one, 1-hydroxy-11-methoxycanthin-6-one, scopoletin, colchicine, NO donors such as pentaerythritol tetranitrate and sydnonimines, S-nitroso derivatives, tamoxifen, staurosporine, β-estradiol, α-estradiol, estriol, estrone, ethinyl estradiol, fosfestrol, medroxyprogesterone, estradiol cypionates, estradiol benzoates, tranilast, kamebakaurin and other terpenoids used for the therapy of cancer, verapamil, tyrosine kinase inhibitors (tyrphostins), cyclosporine A, paclitaxel and derivatives thereof such as 6-α-hydroxy-paclitaxel, baccatin, taxoteres and other macrocyclic oligomers of carbon suboxide (MCS) obtained synthetically and from native sources and derivatives thereof, mofebutazone, acemetacin, diclofenac, lonazolac, dapsone, o-carbamoyl-phenoxy-acetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterol, ademetionine, myrtecaine, polidocanol, nonivamide, levomenthol, benzocaine, aescin, ellipticine, D-24851 (Calbiochem), colcemid, cytochalasin A-E, indanocine, nocodazole, protein S 100, bacitracin, vitronectin receptor antagonists, azelastine, guanidyl cyclase stimulator, tissue inhibitor of metal proteinases 1 and 2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active ingredients from the group of antibiotics such as cefadroxil, cefazolin, cefaclor, cefoxitin, tobramycin, gentamicin, penicillins such as dicloxacillin, oxacillin, sulfonamides, metronidazole, antithrombotics such as argatroban, aspirin, abciximab, synthetic antithrombin, bivalirudin, coumadin, enoxaparin, desulfated and N-reacetylated heparin (Hemoparin^{®}), tissue plasminogen activator, Gpllb/llla platelet membrane receptor, factor Xa-inhibitor antibody, heparin, hirudin, r-hirudin, PPACK, protamine, prourokinase, streptokinase, warfarin, urokinase, vasodilators such as dipyramidole, triazolopyrimidine (Trapidil^{®}), nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65, NF-kB or Bcl-xL antisense oligonucleotides, halofuginone, nifedipine, tocopherol, molsidomine, tea polyphenols, epicatechin gallate, epigallocatechin gallate, boswellic acids and derivatives thereof, leflunomide, anakinra, etanercept, sulfasalazine, tetracycline, triamcinolone, mutamycin, procainimide, retinoic acid, quinidine, disopyramide, flecainide, propafenone, sotalol, amidarone, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprotozoal agents such as chloroquine, mefloquine, quinine, furthermore natural terpenoids such as hippocaesculin, barringtogenol-C21-angelat, 14-dehydroagrostistachin, agroskerin, agrostistachin, 17-hydroxyagrostistachin, ovatodiolides, 4,7-oxycycloanisomelic acid baccharinoids B1, B2, B3 and B7, tubeimoside, bruceanols A, B and C, bruceantinoside C, yadanzioside N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, furthermore cymarin, apocymarin, aristolochic acid, anopterin, hydroxyanopterin, anemonin, protoanemonin, cheliburin chloride, cicutoxin, sinococuline, combrestratin A and B, cudraisoflavone A, curcumin, dihydronitidine, nitidine chloride, 12-β-hydroxypregnadien-3,20-dione, bilobol, ginkgol, ginkgolic acid, helenalin, indicine, indicine-N-oxide, lasiocarpine, glycoside 1a, justicidin A and B, larreatin, malloterin, mallotochromanol, isobutyryl-mallotochromanol, marchantin A, maytansine, lycoridicin, margetin, pancratistatin, aristolactam-All, bisparthenolidine, periplocoside A, deoxypsorospermin, psycorubin, ricin A, sanguinarine, manwu wheat acid, methylsorbifolin, chromones from spathelia, stizophyllin, akagerine, dihydro-usambarensine, hydroxy-usambarine, strychnopentamine, strychnophylline, usambarine, usambarensine, berberine, liriodenine, oxoushinsunine, daphnoretin, lariciresinol, methoxylariciresinol, syringaresinol, umbelliferone, afromoson, acetylvismione B, desacetylvismione A, vismione A and B.

10. Hemocompatible medical product according to claim 8, **characterized in that** the at least one antiproliferative, anti-inflammatory, antiphlogistic and/or antithrombotic agent is selected from the group comprising: paclitaxel and derivatives thereof, β-estradiol, simvastatin, Pl 88 (Progen Ind.), macrocyclic carbon suboxides (MCS) and derivatives thereof, trapidil^{®}, N-(pyridin-4-yl)-[1-4-(4-chlorobenzyl)-indol-3-yl]-glyoxyl amid (D-24851), activated protein C (aPC), Ac-YVAD-CMK, anginex (β-Pep25), Neovastat^{®}, cryptophycin 52, tacrolimus.

11. Hemocompatible medical product according to any one of the preceding claims, **characterized in that**, if multilayer systems are concerned, the at least two layers including and/or not including at least one added hydrophilic polymer contain at least one active ingredient in identical or different concentrations which active agent is bound covalently and/or adhesively.

12. Hemocompatible medical product according to claim 11, **characterized in that**, if multilayer systems are concerned, the last layer is a layer composed of the active ingredient only, wherein said active ingredient is covalently and/or adhesively bound.

13. Hemocompatible medical product according to any one of the preceding claims, **characterized in that** the at least one biostable polysulfone layer containing at least one active ingredient and/or covered with at least one active ingredient is at least partially covered with a biodegradable polymer layer covalently and/or adhesively containing no or at least one active ingredient in identical or different concentrations.

14. Method for biocompatibly and hemocompatibly coating medical products **characterized by** the following steps:
a. providing a stent; and
b. applying at least one biostable layer composed of polysulfone including or not including at least one hydrophilic polymer; and
c. applying and/or inserting at least one antiproliferative, anti-inflammatory, antiphlogistic and/or antithrombotic agent on and/or into the biostable layer; or
b' applying at least one biostable layer composed of polysulfone including or not including the at least one hydrophilic polymer in combination with at least one antiproliferative, anti-inflammatory, antiphlogistic and/or antithrombotic agent; and
c' optionally, applying at least one antiproliferative, anti-inflammatory, antiphlogistic and/or antithrombotic agent on the biostable layer composed of polysulfone;

15. Method according to claim 14 comprising the additional step
d' applying at least one additional layer composed of at least one biodegradable polymer including or not including the insertion and/or application of at least one active ingredient in identical or different concentrations.

16. Hemocompatible medical product which can be obtained by means of a method according to any one of claims 14-15.

17. Hemocompatible medical product according to any one of claims 1- 13 or 16, **characterized in that** the at least one antiproliferative, anti-inflammatory, antiphlogistic and/or antithrombotic agent is released in a controlled manner through the surface coating.

18. Hemocompatible medical product according to any one of claims 1 - 13, 16 or 17, **characterized in that** the respective antiproliferative, anti-inflammatory, antiphlogistic and/or antithrombotic agent is contained in a pharmaceutically active concentration of 0.001 - 10 mg per cm² surface of the medical product and per layer containing the active ingredient.

19. Hemocompatible medical product according to one of claims 1-13 or 16-18, **characterized in that** the medical device is a stent.

## Revendications

1. Produit médical hémocompatible, **caractérisé en ce que** sa superficie est au moins partiellement revêtue d'une couche biologiquement stable composée de polysulfone et **en ce que** au moins un agent actif antiprolifératif, anti-inflammatoire, antiphlogistique et/ ou antithrombotique est présent dans, sous et/ ou sur la au moins une couche biologiquement stable composée de polysulfone.

2. Produit médical hémocompatible selon la revendication 1, **caractérisé en ce que** le polysulfone est sélectionné du groupe comprenant le suivant: polyéthersulfone, polyéthersulfone substitué, polyphénylsulfone, polyphénylsulfone substitué, copolymères séquencés de polysulfones, copolymères séquencés de polysulfones perfluorés, copolymères séquencés de polysulfones semifluorés, copolymères séquencés de polysulfone substitués et/ ou des mélanges des polymères mentionnés ci-dessus.

3. Produit médical hémocompatible selon la revendication 1 ou 2, **caractérisé en ce que** la au moins une couche biologiquement stable composée de polysulfone contient au moins un polymère hydrophile.

4. Produit médical hémocompatible selon la revendication 3, **caractérisé en ce que** le polysulfone et le au moins un polymère hydrophile sont présents dans une proportion de mélange de 50 % en poids : 50 % en poids jusqu'à 99,999 % en poids : 0,001 % en poids.

5. Produit médical hémocompatible selon la revendication 3 ou 4, **caractérisé en ce que** le polymère hydrophile est sélectionné du groupe comprenant le suivant: pyrrollidone de polyvinyle, glycérol, polyéthylène glycol, polypropylène glycol, alcool de polyvinyle, polyhydroxyéthylméthacrylate, polyacrylamide, polyvalérolactone, poly-ε-décalactone, polyacide lactonique, acide polyglycolique, polylactides, polyglycolides, copolymères des polylactides et polyglycolides, poly-ε-caprolactone, poly(hydroxy acide butyrique), polyhydroxybutyrate, polyhydroxyvalérate, polyhydroxybutyrate-co-valérate, poly(1,4-dioxane-2,3-dione), poly(1,3-dioxane-2-one), poly-para-dioxanone, polyanhydrides comme des anhydrides de l'acide polymaléique, fibrine, polycyanoacrylate, polycaprolactone diméthylacrylate, acide β polymaléique, polycaprolactone butylacrylate, polymères séquencés multiples d'oligocaprolactone diols et oligodioxanone diols, polymères séquencés multiples de PEG et poly(butylène térérphtalate), polypivotolactone, triméthyl carbonates de l'acide polyglycolique, polycaprolactone-glycolides, poly(γ-éthyl glutamate), poly(DTH-iminocarbonate), poly(DTE-co-DT-carbonate), poly(bisphénol A-iminocarbonate), polyorthoesters, poly(triméthyl carbonate), poly(iminocarbonate), poly(N-vinyle)-pyrrolidone, alcools de polyvinyle, polyesteramide, polyesters glycolés, poly(phosphoesters), polyphosphazènes, poly[p-carboxyphénoxy)propane], poly(hydroxy acide valérique), polyanhydrides, oxyde de polyéthylène-oxyde de propylène, polyuréthanes souples, polyuréthanes ayant un résidu d'acide aminé dans le tronc, polyétheresters, oxyde de polyéthène, poly(alcène oxalate) polyorthoesters ainsi que leurs copolymères, lipides, carraghénanes, fibrinogène, amidon, collagène, polymères à base de protéines, acides polyaminés, acides polyaminés synthétiques, zéine, zéine modifiée, polyhydroxyalcanoate, acide pectinique, acide actique, fibrine et caséine modifiées et non modifiées, sulfate de carboxyméthyle, albumine, acide hyaluronique, chitosane et ses dérivés, sulfate de chondroïtine, dextrane, β-cyclodextrine et copolymères avec PEG et polypropylène glycol, gomme arabique, guar, gelatine, collagène-N-hydroxy succinimide, phospholipides, modifications et copolymères et/ ou mélanges des substances mentionnées ci-dessus.

6. Produit médical hémocompatible selon la revendication 5, **caractérisé en ce que** le polymère hydrophile est sélectionné du groupe comprenant le suivant: pyrrollidone de polyvinyle, polyéthylène glycol, polypropylène glycol et/ ou glycérol.

7. Produit médical hémocompatible selon l'une quelconque des revendications précédentes, **caractérisé en ce que** le revêtement de la superficie du produit médical se compose de un, deux, trois ou plus couches.

8. Produit médical hémocompatible selon l'une quelconque des revendications précédentes, **caractérisé en ce que** sous et/ ou sur la au moins une couche biologiquement stable composée de polysulfone avec ou sans le au moins un polymère hydrophile est présente au moins une couche de héparine entièrement désulfatée et N-reacétylée, de héparine désulfatée et N-reacétylée, de chitosane N-carboxyméthylé et/ ou partiellement N-acetylé et/ ou de mélanges desdites substances.

9. Produit médical hémocompatible selon l'une quelconque des revendications précédentes, **caractérisé en ce que** le au moins un agent actif antiprolifératif, anti-inflammatoire, antiphlogistique et/ ou antithrombotique est sélectionné du groupe comprenant le suivant : sirolimus (rapamycine), everolimus, somatostatine, tacrolimus, roxithromycine, daunamycine, ascomycine, bafilomycine, erythromycine, midecamycine, josamycine, concanamycine, clarithromycine, troléandomycine, folimycine, cerivastatine, simvastatine, lovastatine, fluvastatine, rosuvastatine, atorvastatine, pravastatine, pitavastatine, vinblastine, vincristine, vindesine, vinorelbine, teniposide, nimustine, carmustine, lomustine, cyclophosphamide, peptide natriurétique de type C (CNP), 4-hydroxycyclophosphamide, estramustine, melphalan, ifosfamide, trofosfamide, chlorambucil, bendamustine, busulfan, procarbazine, treosulfan, temozolomide, thiotépa, daunorubicine, doxorubicine, aclarubicine, épirubicine, mitoxantrone, idarubicine, bléomycine, mitomycine, dactinomycine, méthotrexate, fludarabine, fludarabine-5'-dihydrogènophosphate, cladribine, mercaptopurine, thioguanine, cytarabine, fluorouracil, gemcitabine, capecitabine, docétaxel, carboplatine, cisplatine, cryptophycine, oxaliplatine, amsacrine, irinotécan, topotécan, hydroxycarbamide, miltéfosine, pentostatine, aldesleukine, trétinoïne, asparaginase, pegaspargase, anastrozole, exémestane, létrozole, formestane, aminoglutéthimide, adriamycine, azithromycine, spiramycine, cepharanthine, inhibiteur 2ω de la prolifération de cellules musculaires lisses, épothilone A et B, azathioprine, mycophénolate mofétil, antisens c-myc, antisens b-myc, acide bétulinique, camptothécine, lapachol, β-lapachone, podophyllotoxine, bétuline, 2-ethylhydrazide de l'acide podophyllique, molgramostime (rhuGM-CSF), peginterféron α-2b, lénograstime (r-HuG-CSF), filgrastim, macrogol, anginex, dacarbazine, basiliximab, daclizumab, sélectine (cytokine antagoniste), inhibiteur de la CETP, cadhérine, inhibiteurs de la cytokine, inhibiteur de COX-2, AE-941 (Neovastat^{®}), NFkB, angiopeptine, ciprofloxacine, fluroblastine, anticorps monoclonaux inhibitant la prolifération de cellules musculaires, antagonistes bFGF, probucol, prostaglandine, Ac-YVAD-CMK, 1,11-diméthoxycanthin-6-one, 1-hydroxy-11-méthoxycanthin-6-one, scopolétine, colchicine, donneurs de NO comme le pentaerythrityl tetranitrate et les sydnonimines, dérivés de S-Nitroso, tamoxifène, staurosporine, β-estradiol, α-estradiol, estriol, estrone, éthinylestradiol, fosfestrol, médroxyprogestérone, cipionate d'estradiol, benzoate d'estradiol, kamebakaurine et autres terpénoïdes utilisés dans le traitement anticancéreux, vérapamil, inhibiteurs de la tyrosine kinase (tyrphostine), cyclosporine A, paclitaxel et ses dérivés comme le 6-α-hydroxy-paclitaxel, baccatine, taxotères et autres, oligomeres macrocycliques du sous-oxyde de carbone (MCS) et ses dérivés produits préparés synthétiquement ou obtenus de sources natives, mofebutazone, acémétacine, diclofénac, lonazolac, dapsone, o-carbamoyl-acide phénoxyacétique, lidocaïne, ketoprofène, acide méfénamique, piroxicam, meloxicam, chloroquine phosphate, penicillamine, hydroxychloroquine, auranofine, aurothiomalate de sodium, oxacéprol, célécoxib, β-sitostérol, ademetionine, myrtecaine, polidocanol, nonivamide, lévomenthol, benzocaïne, aescine, ellipticine, D-24851 (Calbiochem), colcemide, cytochalasine A - E, indanocine, nocodazole, protéine S 100, bacitracine, antagonistes du récepteur de la vitronectine, azélastine, stimulateur de la guanidyl cyclase, inhibiteur tissulaire des métalloprotéinases 1 et 2, acides nucléiques libres, acides nucléiques incorporés dans des vecteurs viraux, fragments de l'ADN et l'ARN, inhibiteur activateur plasminogène 1, inhibiteur activateur plasminogène 2, oligonucléotides antisens, inhibiteurs de VEGF, IGF-1, agents actifs du groupe des antibiotiques comme céfadroxil, céfazoline, céfaclor, céfoxitine, tobramycine, gentamycine, pénicillines comme dicloxacilline, oxacilline, sulfonamides, métronidazole, antithrombotiques comme argatroban, aspirine, abciximab, antithrombine synthétique, bivalirudine, coumadine, enoxaparine, héparine desulfatée et N-reacetylée (Hemoparin^{®}), activateur tissulaire plasminogène, récepteur membrane plaquettaire GpIIb/IIIa, anticorps contre l'inhibiteur du facteur Xₐ, héparine, hirudine, r-hirudine, PPACK, protamine, prourokinase, streptokinase, warfarine, urokinase, vasodilatateurs comme dipyridamole, triazolopyrimidine (Trapidil^{®}), nitroprusside, antagonistes de PDGF comme triazolopyrimidine et seramine, inhibiteurs de l'ECA comme captopril, cilazapril, lisinopril, enalapril, losartan, inhibiteurs de la thioprotease, prostacycline, vapiprost, interferôn α, β et γ, antagonistes de l'histamine, bloqueurs de la sérotonine, inhibiteurs de l'apoptose, régulateurs de l'apoptose comme p65, oligonucléotides antisens NF-kB ou Bcl-xL, halofuginone, nifédipine, tocophérol, tranilast, molsidomine, polyphénoles du thé, épicatéchine gallate, épigallocatéchine gallate, acides boswelliques et leurs dérivés, léflunomide, anakinra, etanercept, sulfasalazine, étoposide, tetracycline, triamcinolone, mutamycin, procaïnamide, acide rétinoïque, quinidine, disopyramide, flecaïnide, propafénone, sotalol, amiodarone, stéroïdes naturels ou préparés synthétiquement comme bryophylline A, inotodiol, maquiroside A, ghalakinoside, mansonine, strebloside, hydrocortisone, bétaméthasone, dexamethasone, substances non-stéroïdiens (NSAIDS) comme fénoprofène, ibuprofène, indométhacine, naproxène, phénylbutazone et d'autres agents antiviraux comme acyclovir, ganciclovir et zidovudine, antimycotiques comme clotrimazole, flucytosine, griséofulvine, kétoconazole, miconazole, nystatine, terbinafine, agents antiprotozoaires comme chloroquine, mefloquine, quinine, en outre terpénoïdes naturels comme hippocaesculine, barringtogenol-C21-angélate, 14-dehydroagrostistachine, agroskerine, agrostistachine, 17-hydroxyagrostistachine, ovatodiolides, acide 4,7-oxycyclo anisomélique, baccharinoides B1, B2, B3 et B7, tubeimoside, bruceanoles A, B et C, bruceantinoside C, yadanzioside N et P, isodéoxyelephantopine, tomenphantopine A et B, coronarine A, B, C et D, acide ursolique, acide hyptatique A, zéorine, iso-iridogermanal, maytenfoliol, effusantine A, excisanine A et B, longikaurine B, sculponéatine C, kamebaunine, leukamenine A et B, 13,18-dehydro-6-alpha-senecioyloxy chaparrine, taxamairine A et B, regenilol, triptolide, en outre cymarine, apocymarine, acide aristolochique, aminoptérine, hydroxy aminoptérine, anémonine, proto-anémonine, berbérine, chloride de cheliburine, cicutoxine, sinococuline, combretastatine A et B, curdraisoflvaone A, curcumine, dihydronitidine, chloride de nitidine, 12-béta-hydroxy-pregnadiène-3,20-dione, bilobol, ginkgol, acide ginkgolique, helenaline, indicine, indicine-N-oxyde, lasiocarpine, glycoside 1α, justicidine A et B, larréatine, malloterine, mallotochromanol, isobutyryl-mallotochromanol, marchantine A, maytansine, lycoridicine, margetine, pancratistatine, liriodenine, oxoushinsunin, aristolactam All, bisparthenolidine, periplocoside A, deoxypsorospermine, psycorubine, ricine A, sanguinarine, acide Manwuweiz, méthylsorbifoline, chromones de spathelia, stizophylline, akagerine, dihydrousambaraensine, hydroxyusambarine, strychnopentamine, strychnophylline, usambarine, usambarensine, daphnoretine, lariciresinol, méthoxylariciresinol, syringaresinol, umbelliferone, afromosone, acétyle vismione B, dé-acetyle vismione A, vismione A et B.

10. Produit médical hémocompatible selon la revendication 8, **caractérisé en ce que** le au moins un agent actif antiprolifératif, anti-inflammatoire, antiphlogistique et/ ou antithrombotique est sélectionné du groupe comprenant le suivant : paclitaxel est ses dérivés, β-estradiol, simvastatine, PI 88 (Progen. Ind.), sous-oxydes de carbone macrocycliques (MCS) et ses dérivés, trapidil^{®}, N-(pyridine-4-yl)-[1-4-(4-chlorobenzyle)-indol-3-yl]-glyoxylamide (D-24851), protéine C activée (PCa), Ac-YVAD-CMK, anginex (β-Pep25), neovastat^{®}, cryptophycine 52, tacrolimus.

11. Produit médical hémocompatible selon l'une quelconque des revendications précédentes, **caractérisé en ce que** dans des systèmes multicouches les au moins deux couches avec ou/ et sans au moins un polymère hydrophile ajouté contiennent au moins un agent actif lié de manière covalente et/ ou adhésive dans une concentration d'agent actif identique ou différente.

12. Produit médical hémocompatible selon la revendication 11, **caractérisé en ce que** dans des systèmes multicouches la dernière couche est une couche se composant seulement d'agent actif où ledit agent actif est lié de manière covalente et/ ou adhésive.

13. Produit médical hémocompatible selon l'une quelconque des revendications précédentes, **caractérisé en ce que** la au moins une couche biologiquement stable composée de polysulfone contenant au moins un agent actif et/ ou recouverte d'au moins un agent actif est au moins partiellement recouverte d'une couche polymère biodégradable contenant de manière covalente et/ ou adhésive aucun ou au moins un agent actif dans une concentration identique ou différente.

14. Procédé pour le revêtement biocompatible et hémocompatible de produits médicaux, **caractérisé par** les étapes suivantes :
a. mettre à disposition un stent, et
b. appliquer au moins une couche biologiquement stable composée de polysulfone avec ou sans au moins un polymère hydrophile, et
c. appliquer et/ ou introduire au moins un agent actif antiprolifératif, anti-inflammatoire, antiphlogistique et/ ou antithrombotique sur et/ ou dans la couche biologiquement stable,
ou
b'. appliquer au moins une couche biologiquement stable composée de polysulfone avec ou sans le au moins un polymère hydrophile en combinaison avec un agent actif antiprolifératif, anti-inflammatoire, antiphlogistique et/ ou antithrombotique et
c'. optionnellement, appliquer au moins un agent actif antiprolifératif, anti-inflammatoire, antiphlogistique et/ ou antithrombotique sur la couche biologiquement stable composée de polysulfone.

15. Procédé selon la revendication 14 comprenant l'étape supplémentaire
d'. appliquer au moins une couche supplémentaire composée d'au moins un polymère biodégradable sans ou avec l'introduction et/ ou l'application d'au moins un agent actif dans une concentration identique ou différente.

16. Produit médical hémocompatible disponible conformément au procédé selon l'une quelconque des revendications 14 ou 15.

17. Produit médical hémocompatible selon l'une quelconque des revendications 1 à 13 ou 16, **caractérisé en ce que** le au moins un agent actif antiprolifératif, anti-inflammatoire, antiphlogistique et/ ou antithrombotique est libéré de manière contrôlée à travers le revêtement de la surface.

18. Produit médical hémocompatible selon l'une quelconque des revendications 1 à 13, 16 ou 17, **caractérisé en ce que** l'agent actif antiprolifératif, anti-inflammatoire, antiphlogistique et/ ou antithrombotique respectif est contenu dans une concentration pharmaceutiquement active de 0,001- 10 mg par cm² superficie du produit médical et par couche contenant l'agent actif.

19. Produit médical hémocompatible selon l'une quelconque des revendications 1 à 13 ou 16 à 18, **caractérisé en ce que** le produit médical est un stent.
